# EUROPÄISCHE PATENTSCHRIFT

(11) **EP 3 630 266 B1**
(45) Veröffentlichungstag und Bekanntmachung des Hinweises auf die Patenterteilung: **23.06.2021**
(21) Anmeldenummer: 18730250.0
(22) Anmeldetag: 23.05.2018
(51) Int. Cl.: A61N 1/05, H01R 13/00

(54) **ELEKTROMEDIZINISCHER ADAPTER, ELEKTROMEDIZINISCHE ELEKTRODE UND ELEKTROMEDIZINISCHER IMPULSGEBER**
ELECTROMEDICAL ADAPTER, ELECTROMEDICAL ELECTRODE AND ELECTROMEDICAL PULSE GENERATOR
ADAPTATEUR ÉLECTRO-MÉDICAL, ÉLECTRODE ÉLECTRO-MÉDICALE ET ÉMETTEUR D'IMPULSIONS ÉLECTRO-MÉDICAL

(30) Priorität: 23.05.2017 DE 102017111280
(43) Veröffentlichungstag der Anmeldung: 08.04.2020
(73) Patentinhaber: Osypka AG, 79618 Rheinfelden-Baden (DE)
(72) Erfinder: GÖTTSCHE, Thorsten, 79618 Rheinfelden (DE); GRÄFE, Maik, 79618 Rheinfelden (DE)
(74) Vertreter: Mertzlufft-Paufler, Cornelius
(86) Internationale Anmeldenummer: PCT/EP2018/063564
(87) Internationale Veröffentlichungsnummer: WO 2018/215564

(56) Entgegenhaltungen:
- WO-A1-00/51489
- DE-A1- 4 119 222
- GB-A- 2 456 441
- US-A1- 2010 057 175
- US-B2- 7 292 894

## Beschreibung

Die Erfindung betrifft einen elektromedizinischen Adapter zur elektrischen Verbindung eines gewendelten Elektrodenabschnitts mit einem ungewendelten Elektrodenabschnitt einer elektromedizinischen Elektrode.

Die Erfindung betrifft ferner eine elektromedizinische Elektrode, insbesondere eine implantierbare elektromedizinische Elektrode, sowie einen elektromedizinischen Impulsgeber, der eine elektromedizinische Elektrode zur Abgabe elektromedizinischer Stimulationsimpulse auf einen Patienten aufweist. Der Impulsgeber kann dabei ein elektromedizinisches Stimulationsgerät sein, insbesondere ein implantierbares elektromedizinisches Stimulationsgerat.

Derartige Elektroden und elektromedizinische Impulsgeber sind aus dem Stand der Technik in unterschiedlichen Ausführungsformen bekannt.

Hierbei kommen unterschiedliche Elektrodenformen zum Einsatz. So offenbart beispielsweise die Druckschrift US 2010/0057175 A1 eine implantierbare elektromedizinische Leitung, die eine erste Leitungsbaugruppe mit einer distalen Spitze und einem medialen Ende umfasst. Dabei weist die erste Leitungsbaugruppe eine Vielzahl von außeren Kontakten und mindestens einen leitfähigen Draht auf. Der mindestens eine leitfähige Draht erstreckt sich von mindestens einem außeren Kontakt in Richtung auf das mediale Ende der ersten Leitungsbaugruppe. Ferner umfasst die vorbekannte Leitung einen Mittelabschnitt mit einem ersten Ende und einem zweiten Ende, wobei der Mittelabschnitt eine Vielzahl von Leitern umfasst, die sich vom ersten Ende zum zweiten Ende erstrecken. Des Weiteren umfasst die Leitung eine Zwischenbaugruppe, die zwischen der ersten Leitungsbaugruppe und dem ersten Ende des Mittelabschnitts angeordnet ist. Die erste Zwischenbaugruppe hat mehrere leitende Elemente, wobei mindestens eines der leitfähigen Elemente dazu eingerichtet ist, den mindestens einen leitfähigen Draht der ersten Leitungsbaugruppe mit mindestens einem der Leiter am ersten Ende des Mittelabschnitts elektrisch zu koppeln.

Insbesondere dort, wo eine hohe Flexibilität und große Bruchresistenz der Elektroden von Interesse ist, kommen so genannte gewendelte Elektroden zum Einsatz, die zumindest einen gewendelten, also in einer Schraubenlinie gewickelten elektrischen Leiter aufweisen.

Solche gewendelten Elektroden können vor allem in stark bewegte Körperregionen implantiert werden, beispielsweise im Übergangsbereich zwischen Rumpf und Kopf eines Patienten. Sie eignen sich dazu, da sie Bewegungen und Wechselbelastungen vergleichsweise gut tolerieren, ohne zu brechen.

Die durch die Wicklung oder Wendelung der elektrischen Leiter dieser Elektroden gewonnene Flexibilität geht allerdings mit einer größeren Leiterlänge einher, als sie bei ungewendelten Elektroden mit ungewendelten, gerade verlaufenden elektrischen Leitern notwendig ist. Da der elektrische Widerstand der Elektrode und ihrer Leiter mit der Länge der Leiter ansteigt, haben gewendelte Elektroden im Vergleich zu ungewendelten Elektroden mit ungewendelten elektrischen Leitern einen höheren elektrischen Widerstand. Zudem sind sie aufgrund ihrer Wicklung oder Wendelung aufwendiger und damit teurer in der Herstellung. Ungewendelte Elektroden haben im Vergleich dazu einen geringeren elektrischen Widerstand und lassen sich günstiger herstellen, sind jedoch nicht so flexibel und bruchresistent wie gewendelte Elektroden.

Aufgabe der Erfindung ist es, einen elektromedizinischen Adapter sowie eine elektromedizinische Elektrode der eingangs genannten Art bereitzustellen, die die Herstellung, Handhabung und Verwendung solcher Elektroden vereinfachen.

Diese Aufgabe wird durch die Gegenstände der unabhängigen Anspruche gelöst.

Zur Lösung der Aufgabe wird insbesondere ein medizinischer Adapter mit den Merkmalen des unabhängigen, auf einen elektromedizinischen Adapter gerichteten Anspruchs vorgeschlagen. Insbesondere wird zur Lösung dieser Aufgabe ein elektromedizinischer Adapter zur elektrischen Verbindung eines gewendelten Elektrodenabschnitts mit einem ungewendelten Elektrodenabschnitt einer elektromedizinischen Elektrode vorgeschlagen, der wenigstens ein Kontaktelement aufweist, das mit einem ungewendelten Leiter des ungewendelten Elektrodenabschnitts verbindbar und derart an einem Grundkörper des Adapters angeordnet ist, dass ein in Gebrauchsstellung das wenigstens eine Kontaktelement kontaktierender gewendelter Elektrodenabschnitt eine Längsachse des Grundkörpers des Adapters mit seinem wenigstens einen gewendelten Leiter umschließt. Vorzugsweise weist der Adapter mehrere Kontaktelemente auf, die vorzugsweise gleichmäßig an dem Grundkörper, insbesondere gleichmäßig verteilt um die Längsachse, des Adapters verteilt angeordnet sein können. Auf diese Weise stehen mehrere Kontaktelemente des Adapters für den gewendelten Elektrodenabschnitt zur Verfügung. So kann sichergestellt werden, dass zumindest wenigstens ein gewendelter Leiter des gewendelten Elektrodenabschnitts eines der Kontaktelemente kontaktiert, wenn der gewendelte Elektrodenabschnitt die Längsachse in seiner Gebrauchsstellung an dem Adapter umschließt.

Der wenigstens eine gewendelte Leiter kann die Längsachse des Grundkörpers des Adapters radial umgeben und in Gebrauchsstellung wenigstens ein Kontaktelement des Adapters berühren und damit elektrisch kontaktieren. Die zuvor erwähnte Längsachse des Grundkörpers des Adapters kann eine Längsmittelachse des Grundkörpers des Adapters sein. Auf diese Weise wird einen Adapter bereitgestellt, mit dem eine ausreichend stabile und vor allem leicht herzustellende elektrische Verbindung zwischen einem ungewendelten Elektrodenabschnitt und einem gewendelten Elektrodenabschnitt einer elektromedizinischen Elektrode hergestellt werden kann. Der Adapter erlaubt es, maßgeschneiderte Elektroden auf einfache Weise herzustellen, die die Vorteile von gewendelten Elektroden und ungewendelten Elektroden miteinander kombinieren.

Bei einer Ausführungsform des elektromedizinischen Adapters kann vorgesehen sein, dass der Grundkörper des Adapters einen Steckbereich aufweist. Zumindest ein Kontaktelement des Adapters kann an einer Außenseite des Steckbereichs angeordnet sein, wo es für eine Kontaktierung zumindest eines gewendelten Leiters des gewendelten Elektrodenabschnitts gut zugänglich ist. Der Adapter kann mit seinem Steckbereich in eine von dem wenigstens einen gewendelten Leiter definierte Innenlängshöhlung in einem Anschlussende des gewendelten Elektrodenabschnitts zur Kontaktierung des gewendelten Elektrodenabschnitts eingesteckt werden. Das Einstecken kann dabei reversibel oder auch irreversibel lösbar erfolgen. Auf diese Weise lässt sich ein zuverlässiger Kontakt zwischen dem wenigstens einen Kontaktelement des elektromedizinischen Adapters und dem wenigstens einen gewendelten Leiter des gewendelten Elektrodenabschnitts herstellen.

Es ist auch möglich, dass der Grundkörper des Adapters eine Buchse zur Aufnahme eines Anschlussendes des gewendelten Elektrodenabschnitts aufweist. Innerhalb der Buchse können das wenigstens eine Kontaktelement oder mehrere Kontaktelemente des Adapters zur Kontaktierung des wenigstens einen gewendelten Leiters angeordnet sein. Die Buchse kann derart ausgestaltet sein, dass sie ein Anschlussende des gewendelten Elektrodenabschnitts in sich aufnehmen kann. Ist das Anschlussende des gewendelten Elektrodenabschnitts in der Buchse des Adapters positioniert, kommt es mit dem entsprechend angeordneten Kontaktelement des elektromedizinischen Adapters in Berührung. Dies so, dass eine elektrische Verbindung zwischen dem wenigstens einen Kontaktelement des elektromedizinischen Adapters und dem wenigstens einen gewendelten Leiter des gewendelten Elektrodenabschnitts hergestellt wird.

Vorteilhaft kann es sein, wenn der Grundkörper des Adapters aus einem elektrisch isolierenden Material besteht. Möglich ist es auch, dass der Adapter an seinem Grundkörper für jedes Kontaktelement eine insbesondere nutförmige Halteaufnahme aufweist. Auf diese Weise können ein oder auch mehrere Kontaktelemente mithilfe der Halteaufnahmen sicher und definiert an dem Grundkörper des Adapters befestigt werden. Hierbei kann ein Kontaktelement in eine Halteaufnahme eingesetzt werden. Je nach Variante des Adapters kann vorgesehen sein, dass wenigstens eine Halteaufnahme an dem Steckbereich des und/oder wenigstens eine Halteaufnahme innerhalb der Buchse angeordnet ist/sind.

Eine besonders einfache Kontaktierung zwischen den Leitern der beiden unterschiedlichen Elektrodenabschnitte mithilfe des Adapters ist möglich, da das wenigstens eine Kontaktelement des Adapters eine Kontakthülse ist. Diese ist in Gebrauchsstellung in eine, beispielsweise die bereits zuvor erwähnte, Halteaufnahme des Adapters eingesetzt. Die Halteaufnahme kann dann auch als Hülsenaufnahme bezeichnet werden. Das wenigstens eine Kontaktelement steht in Gebrauchsstellung radial aus der Halteaufnahme zur Kontaktierung des wenigstens einen gewendelten Leiters eines gewendelten Elektrodenabschnitts nach außen oder nach innen über. Auf diese Weise ist es möglich, das Anschlussende des gewendelten Elektrodenabschnitts mit seinem wenigstens einen gewendelten Leiter unter einer gewissen radialen Pressung, bei einem Adapter mit Buchse entweder radial von innen oder bei einem Adapter mit Steckbereich, an dessen Außenseite ein oder mehrere Kontaktelemente angeordnet sind, radial von außen, mit dem wenigstens einen Kontaktelement in Berührung zu bringen. Zudem kann das wenigstens eine Kontaktelement auf diese Weise so angeordnet sein, dass es für eine elektrische Kontaktierung gut zugänglich ist.

Bei einem Adapter, der, insbesondere an seiner Außenseite seines Steckbereichs, eine Halteaufnahme für ein elektrisches Kontaktelement aufweist, kann diese Halteaufnahme eine stirnseitige Öffnung haben, durch die das in der Halteaufnahme positionierte elektrische Kontaktelement für einen ungewendelten Leiter des ungewendelten Elektrodenabschnitts zugänglich ist. Der ungewendelte Leiter lässt sich durch diese Öffnung führen und mit dem Kontaktelement in der Halteaufnahme in Verbindung bringen.

Hierbei kann die stirnseitige Öffnung so dimensioniert sein, dass durch sie sowohl das als Kontakthülse ausgebildete Kontaktelement als auch der ungewendelte Leiter in die Halteaufnahme einführbar sind.

Eine zuverlässige Kontaktierung eines oder mehrerer gewendelter Leiter eines gewendelten Elektrodenabschnitts ist möglich, wenn der Adapter mehrere, vorzugsweise gleichmäßig um eine Längsachse des Adapters verteilt angeordnete, Kontaktelemente und/oder Halteaufnahmen aufweist. In jeder der Halteaufnahmen kann jeweils ein Kontaktelement angeordnet werden oder sein. Besonders bevorzugt kann der Adapter so viele Halteaufnahmen wie der mit dem Adapter zu verbindende ungewendelte Elektrodenabschnitt ungewendelte Leiter aufweisen. So ist für jeden ungewendelten Leiter des ungewendelten Elektrodenabschnitts jeweils ein Kontaktelement verfügbar.

Wenn das oder die Kontaktelemente als Kontakthülsen ausgebildet sind, ist es möglich, den oder die ungewendelten Leiter durch Einschieben ihrer Leiterenden in die jeweilige Kontakthülse mit dieser elektrisch zu verbinden. Das eingeschobene Leiterende eines ungewendelten Leiters kann mit dem Kontaktelement beispielsweise verschweißt, verlötet, vercrimpt oder auch verklebt oder gebondet, insbesondere durch Drahtbonden befestigt werden.

Weist der Adapter mehrere Halteaufnahmen mit darin befindlichen Kontaktelementen auf, kann ein gewendelter Leiter mehrere Kontaktelemente des Adapters gleichzeitig kontaktieren. Auf diese Weise kann die Sicherheit und Zuverlässigkeit der elektrischen Verbindung zwischen dem Adapter und dem gewendelten Elektrodenabschnitt der elektromedizinischen Elektrode weiter verbessert werden.

Vorteilhaft kann es sein, wenn der Adapter zur reversibel lösbaren Verbindung mit einem gewendelten Elektrodenabschnitt eingerichtet ist. Dann ist ein einfacher Austausch des Elektrodenabschnitts oder auch des Adapters möglich.

Eine gute Anordnung des wenigstens einen gewendelten Leiters an dem ihm zugeordneten Kontaktelement ist möglich, wenn das wenigstens eine Kontaktelement, insbesondere die Kontakthülse, eine Rastnut aufweist. In diese Rastnut kann wenigstens ein gewendelter Leiter des gewendelten Elektrodenabschnitts bei in Gebrauchsstellung an dem Adapter befindlichem gewendelten Elektrodenabschnitt einrasten oder eingerastet sein. Je nach Ausbildung der Rastnut ist so sogar eine haptische Rückmeldung bei der Montage des Adapters an einen gewendelten Elektrodenabschnitt einer elektromedizinischen Elektrode möglich, wenn der gewendelte Leiter in die Rastnut einrastet. Das Einrasten kann zum Beispiel durch eine radial nach außen gerichtete Bewegung des gewendelten Leiters in die Rastnut erfolgen, wenn der Adapter eine Buchse mit darin angeordnetem Kontaktelement aufweist.

Ist der Adapter ein solcher, der über einen Steckbereich verfügt, an dessen Außenseite ein Kontaktelement, insbesondere eine Kontakthülse, mit einer derartige Rastnut vorgesehen ist, kann der zumindest eine gewendelte Leiter des gewendelten Elektrodenabschnitts durch eine radial nach innen gerichtete Bewegung in die Rastnut hineinrutschen und dort einrasten.

Bei einer weiteren Ausführungsform des elektromedizinischen Adapters kann der Grundkörper des Adapters aus einem zu einem Hohlkörper gerollten Adapterabschnitt einer Leiterplattenfolie hergestellt sein. Das wenigstens eine Kontaktelement des elektromedizinischen Adapters kann eine an dem Adapterabschnitt ausgebildete elektrische Kontaktfläche sein. Je nach Wicklungsrichtung oder Rollrichtung zur Herstellung des Hohlkörpers kann das wenigstens eine Kontaktelement dann an einer Innenseite des Hohlkörpers oder an einer Außenseite des Hohlkörpers angeordnet sein. Ist das wenigstens eine Kontaktelement innerhalb des Hohlkörpers angeordnet, kann der Hohlkörper, der den Grundkörper des Adapters bildet, als Buchse zur Aufnahme eines Anschlussendes eines mit dem Adapter zu verbindenden gewendelten Elektrodenabschnitts fungieren.

Der Hohlkörper kann ein zylindrischer Hohlkörper sein, der einen runden oder kreisrunden Querschnitt aufweist. Die Verwendung eines Hohlkörpers mit einem eckigen oder mehreckigen Querschnitt als Grundkörper eines Adapters kann für eine gut zu erreichende Anordnung des wenigstens einen Kontaktelements günstig sein. Insbesondere dann, wenn das wenigstens eine Kontaktelement in einem Bereich einer Ecke oder Kante des eckigen Grundkörpers an der Außenseite des den Grundkörper bildenden Hohlkörpers angeordnet ist, kann es etwas von dem Grundkörper abstehen und zuverlässig kontaktiert werden.

Ist das wenigstens eine Kontaktelement an der Außenseite des Grundkörpers des Adapters angeordnet, fungiert dieser Bereich, in dem die als Kontaktelement wirkende Kontaktfläche des Adapterabschnitts angeordnet ist, als Steckbereich. Mit diesem Steckbereich kann der Adapter in eine Innenlängshöhlung an einem Anschlussende eines gewendelten Elektrodenabschnitts eingesteckt werden.

Bei einem Grundkörper, der aus einem Adapterabschnitt einer Leiterplattenfolie hergestellt ist, der zu einem zylindrischen Hohlkörper gerollt Adapterabschnitt ist und der wenigstens ein innenliegendes Kontaktelement aufweist, kann es vorteilhaft sein, wenn der Adapterabschnitt der Leiterplattenfolie mit zumindest einem Durchbruch versehen ist. Dieser Durchbruch kann als Fenster dienen, durch das die wenigstens eine Kontaktfläche, insbesondere wenn sie innerhalb der Buchse angeordnet ist, von außen zugänglich ist. Auf diese Weise kann ein gewendelter Leiter eines gewendelten Elektrodenabschnitts zum Beispiel durch Schweißen, Löten oder Kleben oder auch Bonden, insbesondere Drahtbonden auch innerhalb der so definierten Buchse des elektromedizinischen Adapters durch den Durchbruch hindurch fest mit dem Kontaktelement verbunden werden. Für das Drahtbonden kann es vorteilhaft sein, wenn ein mit Drahtbonden zu verbindendes Leiterende des wenigstens einen gewendelten Leiters abgeflacht ist. Das Abflachen der Leiterenden kann beispielsweise durch Walzen oder Hämmern erfolgen.

Bei allen Ausführungsformen des elektromedizinischen Adapters kann es vorteilhaft sein, wenn der Grundkörper des Adapters zylindrisch ist. Auf diese Weise lässt sich der Grundkörper des Adapters zum Beispiel mit seinem Steckbereich bestmöglich und passgenau in eine Innenlängshöhlung eines Anschlussendes eines gewendelten Elektrodenabschnitts einschieben. Wenn der zylindrische Grundkörper des Adapters eine zylindrische Buchse aufweist, lässt sich das Anschlussende des gewendelten Elektrodenabschnitts passgenau mit der Innenwandung der Buchse und/oder darin angeordneten Kontaktelementen zur Anlage bringen. Ferner kann ein Adapter mit einem zylindrischen Grundkörper einen stufenlosen Übergang zwischen den beiden Elektrodenabschnitten ermöglichen. Dies insbesondere dann, wenn ein maximaler Außendurchmesser des Adapters auf die Außendurchmesser der beiden Elektrodenabschnitte abgestimmt ist.

Günstig kann es sein, wenn der Steckbereich des Adapters einen geringeren Durchmesser als der übrige Grundkörper aufweist. So kann der Steckbereich des Adapters abgesetzt sein und das auf den Steckbereich aufgeschobene Anschlussende des gewendelten Elektrodenabschnitts zusammen mit dem Adapter eine stufenlose Außenfläche bilden. Hierbei kann sich ein stufenloser Übergang zwischen dem gewendelten Elektrodenabschnitt, dem Adapter und dem ungewendelten Elektrodenabschnitt ergeben.

Der Grundkörper des Adapters kann einen runden oder kreisrunden Querschnitt aufweisen. Dies insbesondere in seinem Steckbereich. Zudem kann der Grundkörper an einem freien Ende des Steckbereichs eine Anfasung oder eine Abrundung aufweisen zum erleichterten Einstecken oder Einführen des Adapters in eine Innenlängshöhlung eines Anschlussendes eines gewendelten Elektrodenabschnitts. Grundsätzlich kann der Grundkörper des Adapters auch einen eckigen oder mehreckigen Querschnitt aufweisen. Dies auch in seinem Steckbereich.

Um den Adapter an einem ungewendelten Elektrodenabschnitt festlegen zu können, kann der Adapter an seinem Grundkörper ein zu einem Gegensteckelement eines ungewendelten Elektrodenabschnitts korrespondierendes Steckelement aufweisen. So kann der Adapter in Gebrauchsstellung über eine Steckverbindung mit dem ungewendelten Elektrodenabschnitt verbunden sein. Auf diese Weise wird eine Verbindung des Adapters mit dem ungewendelten Elektrodenabschnitt stabilisiert und kann so auch größeren Belastungen standhalten.

Die zuvor genannte Aufgabe wird bei einer medizinischen Elektrode der eingangs genannten Art mit den Mitteln und Merkmalen des auf eine solche Elektrode gerichteten unabhängigen Anspruchs gelöst. Insbesondere wird zur Lösung dieser Aufgabe eine elektromedizinische Elektrode mit wenigstens einem gewendelten Elektrodenabschnitt und wenigstens einem ungewendelten Elektrodenabschnitt vorgeschlagen, wobei der gewendelte Elektrodenabschnitt zumindest einen gewendelten elektrischen Leiter und der ungewendelte Elektrodenabschnitt zumindest einen ungewendelten elektrischen Leiter umfassen, und wobei die beiden Elektrodenabschnitte über einen Adapter elektrisch miteinander verbunden sind.

Auf diese Weise wird eine elektromedizinische Elektrode geschaffen, die die Vorteile der beiden unterschiedlichen Elektrodenarten miteinander verbindet. So ist es möglich, den ungewendelten Elektrodenabschnitt der elektromedizinischen Elektrode zum Beispiel in einem Körperteil zu verlegen, der eher weniger stark bewegt ist, so dass hier der ungewendelte Elektrodenabschnitt vergleichsweise geringen Belastungen ausgesetzt wird, die von ihm toleriert werden. Insbesondere in solchen Körperabschnitten, die vergleichsweise stark bewegt werden, kann der gewendelte und dadurch flexiblere Elektrodenabschnitt verlegt werden. Die so geschaffene elektromedizinische Elektrode ist ausreichend flexibel und resistent gegen Bruch durch Wechselbelastungen und hat trotzdem einen geringeren elektrischen Widerstand als solche Elektroden, die ausschließlich aus gewendelten Leitern hergestellt sind. Besonders zuverlässig und stabil lässt sich die Verbindung zwischen den beiden Elektrodenabschnitten gestalten, wenn als Adapter ein Adapter nach einem der Ansprüche 1 bis 8 verwendet wird.

Wenn der gewendelte Elektrodenabschnitt reversibel lösbar mit dem Adapter verbindbar und in Gebrauchsstellung verbunden ist, lässt sich der gewendelte Elektrodenabschnitt bei Bedarf austauschen und durch einen anderen ersetzen. Auch ein Austausch des Adapters kann so ermöglicht werden.

Bei einer Ausführungsform der erfindungsgemäßen Elektrode kann der zumindest eine gewendelte Leiter des gewendelten Elektrodenabschnitts den Adapter zur Kontaktierung des ungewendelten Elektrodenabschnitts außenseitig übergreifen. Hierbei kann der zumindest eine gewendelte Leiter des gewendelten Elektrodenabschnitts einen oder den Steckbereich des Adapters zur Kontaktierung des ungewendelten Elektrodenabschnitts außenseitig übergreifen. Hier fungiert der Adapter somit als Einsteckteil, das in eine von dem gewendelten Leiter des gewendelten Elektrodenabschnitts definierte Innenlängshöhlung eingesteckt werden kann, um einen elektrischen Kontakt zwischen dem gewendelten Leiter und dem ungewendelten Leiter des ungewendelten Elektrodenabschnitts herzustellen. Eine Montage der Elektrode wird so vereinfacht.

Es ist aber auch möglich, dass der gewendelte Elektrodenabschnitt zur Kontaktierung des ungewendelten Elektrodenabschnitts mit seinem Anschlussende in eine oder die Buchse des Adapters eingesteckt ist. Hierbei wird ausgenutzt, dass der Adapter eine Buchse für den zumindest einen gewendelten Leiter aufweist, in die der gewendelte Elektrodenabschnitt mit einem Ende zur Kontaktierung des gewendelten Elektrodenabschnitts einsteckbar und in Gebrauchsstellung eingesteckt ist.

Wie bereits zuvor ausgeführt wurde, kann der Adapter zumindest ein Kontaktelement aufweisen, über das der zumindest eine gewendelte Leiter des gewendelten Elektrodenabschnitts mit dem wenigstens einen ungewendelten Leiter des ungewendelten Elektrodenabschnitts elektrisch verbindbar und in Gebrauchsstellung verbunden ist. Dabei kann das zumindest eine Kontaktelement an einer Außenseite eines Steckbereichs des Adapters und damit von außen für den gewendelten Elektrodenabschnitt zugänglich angeordnet sein. Es ist auch möglich, dass das zuvor erwähnte oder ein weiteres Kontaktelement innerhalb der Buchse des Adapters angeordnet wird und/oder angeordnet ist. Somit ist das Kontaktelement auch für einen gewendelten Elektrodenabschnitt zur Kontaktierung zugänglich, der in die Buchse des Adapters eingesteckt wird.

Bei einer Variante der elektromedizinischen Elektrode ist vorgesehen, dass der ungewendelte Elektrodenabschnitt von einem Lumenschlauch mit zumindest einem inneren Lumen und zumindest einem darin angeordneten ungewendelten Leiter gebildet ist. Vorzugsweise kann als ungewendelter Elektrodenabschnitt auch ein Mehrlumenschlauch verwendet werden, der mehrere innere Lumen aufweist, in denen jeweils ein ungewendelter Leiter angeordnet ist.

Der Adapter kann aus einem elektrisch isolierenden Material bestehen und an seiner Außenseite seines Steckbereichs zumindest eine Halteaufnahme für das wenigstens eine Kontaktelement aufweisen, in die das Kontaktelement eingesetzt ist. Auf diese Weise ist es möglich, das wenigstens eine Kontaktelement an der Außenseite des Steckbereichs des Adapters festzulegen und so einen zuverlässigen Anschluss des Adapters an den gewendelten Elektrodenabschnitt zu ermöglichen.

Bei der Verwendung eines ungewendelten Elektrodenabschnitts, der einen Lumenschlauch umfasst, ist es möglich, dass jeder ungewendelte Leiter des ungewendelten Elektrodenabschnitts mit einem aus dem Lumenschlauch ragenden Leiterende mit einem, vorzugsweise mit jeweils einem, Kontaktelement des Adapters verbunden ist. Die Verbindung des jeweiligen Leiterendes mit dem Kontaktelement kann hierbei zum Beispiel durch Crimpen, durch Löten, durch Schweißen, durch Bonden, durch Drahtbonden, durch Federkraft und/oder durch Kleben erfolgen. Eine Verbindung des jeweiligen Leiterendes mit dem Kontaktelement durch Crimpen ist besonders dann möglich, wenn das Kontaktelement als Kontakthülse ausgebildet ist. Auf diese Weise kann das jeweilige Leiterende des ungewendelten Leiters zunächst in das Innere der Kontakthülse eingeschoben und anschließend durch Vercrimpen der Kontakthülse mit dieser verbunden werden.

Der wenigstens eine gewendelte Leiter des gewendelten Elektrodenabschnitts kann durch Schweißen, Kleben, Löten und/oder Bonden oder Drahtbonden mit dem wenigstens einen Kontaktelement verbunden sein. Wenn der wenigstens eine gewendelte Leiter des gewendelten Elektrodenabschnitts durch Federkraft mit dem wenigstens einen Kontaktelement verbunden ist, kann der gewendelte Elektrodenabschnitt besonders einfach reversibel lösbar mit dem Adapter verbunden sein.

Wie bereits zuvor ausgeführt wurde, kann der Adapter mehrere, vorzugsweise gleichmäßig um eine Längsachse des Adapters verteilt angeordnete, Kontaktelemente und/oder Halteaufnahmen aufweisen. Dabei kann in jeder der Halteaufnahmen jeweils ein mit einem ungewendelten Leiter verbundenes Kontaktelement angeordnet sein. Vorzugsweise weist der Adapter so viele Halteaufnahmen wie der ungewendelte Elektrodenabschnitt ungewendelte Leiter auf. Auf diese Weise steht für jeden ungewendelten Leiter des ungewendelten Elektrodenabschnitts genau eine Halteaufnahme zur Verfügung, in die ein Kontaktelement, vorzugsweise eine Kontakthülse, einsetzbar ist.

Eine zuverlässige elektrische Verbindung der beiden Elektrodenabschnitte lässt sich erzielen, wenn der wenigstens eine gewendelte Leiter des gewendelten Elektrodenabschnitts jeweils wenigstens zwei Kontaktelemente des Adapters in Gebrauchsstellung kontaktiert. Eine besonders zuverlässige elektrische Verbindung der beiden Elektrodenabschnitte lässt sich erzielen, wenn der wenigstens eine oder jeder gewendelte Leiter des gewendelten Elektrodenabschnitts in Gebrauchsstellung an dem Adapter jedes Kontaktelement des Adapters zumindest einmal kontaktiert. Hierfür kann es vorteilhaft sein, wenn der oder die gewendelten Leiter den Adapter und sein wenigstens ein Kontaktelement in Gebrauchsstellung über einen Winkelbereich von mehr als 180 Grad, besonders bevorzugt von mehr als 360 Grad radial umgeben. Jeder gewendelte Leiter kann in Gebrauchsstellung also wenigstens einmal um den Adapter herumgewickelt sein. So wird jeder gewendelte Leiter in Gebrauchsstellung wenigstens einmal an jedem Kontaktelement des Adapters vorbeigeführt und kann des kontaktieren.

Ein Außendurchmesser, insbesondere des Anschlussendes, des gewendelten Elektrodenabschnitts kann größer als ein Durchmesser eines gedachten Hüllkreises sein, der in Gebrauchsstellung an dem Adapter innerhalb seiner Buchse befindliche Kontaktelemente, insbesondere Kontakthülsen, einhüllt. So kann der gewendelte Elektrodenabschnitt unter radialer Pressung zwischen den Kontaktelementen in der Buchse des Adapters angeordnet sein. Dies begünstigt eine zuverlässige elektrische Verbindung der Elektrodenabschnitte.
Ferner kann ein lichter Innendurchmesser, insbesondere einer Innenlängshöhlung im Bereich eines Anschlussendes, des gewendelten Elektrodenabschnitts kleiner als ein Durchmesser eines gedachten Hüllkreises sein, der in Gebrauchsstellung an einer Außenseite des Grundkörpers des Adapters, insbesondere in seinem Steckbereich, befindliche Kontaktelemente, insbesondere Kontakthülsen, einhüllt. So kann der gewendelte Elektrodenabschnitt den Adapter und seine Kontaktelemente unter radialer Pressung übergreifen. Diese Variante kommt insbesondere dort zur Anwendung, wo ein oder mehrere Kontaktelemente an einer Außenseite eines Steckbereichs des Adapters ausgebildet sind.

Bei beiden zuvor beschriebenen Varianten kann die Pressung derart gewählt sein, dass allein aufgrund der Pressung eine reversibel lösbare oder auch eine permanente Steckverbindung zwischen dem gewendelten Elektrodenabschnitt und dem Adapter herstellbar ist. Denkbar ist es in diesem Zusammenhang, auf weitere Befestigungsmaßnahmen, wie Bonden, Drahtbonden, Kleben, Löten oder Schweißen zu verzichten. Der gewendelte Elektrodenabschnitt kann bei beiden Varianten als Feder wirken und eine radial nach außen oder nach innen wirkende Federkraft zur Erzeugung der vorgenannten Pressung auf den Adapter bringen. Der gewendelte Elektrodenabschnitt kann dann kraftschlüssig mit dem Adapter verbunden sein.

Bei einer Ausführungsform der Erfindung kann der Adapter mit dem ungewendelten Elektrodenabschnitt verbunden werden. Besonders einfach kann diese Verbindung gestaltet sein, wenn der Adapter und der ungewendelte Elektrodenabschnitt zusammensteckbar und in Gebrauchsstellung zusammengesteckt sind. Dabei ist es denkbar, dass der Adapter zum Beispiel mit dem Lumenschlauch des Elektrodenabschnitts zusammengesteckt und gegebenenfalls auch mit diesem verklebt ist.

Zu diesem Zweck kann zwischen dem ungewendelten Elektrodenabschnitt und dem Adapter eine Steckverbindung ausgebildet sein. Bei dieser Steckverbindung kann vorgesehen sein, dass der Adapter ein, vorzugsweise stirnseitig angeordnetes, Steckelement aufweist, mit dem er mit einem korrespondierend gestalteten Gegensteckelement an dem ungewendelten Elektrodenabschnitt, insbesondere an dem Lumenschlauch des ungewendelten Elektrodenabschnitts, zusammengesteckt werden kann und in Gebrauchsstellung zusammengesteckt ist. Nach Herstellen der Steckverbindung kann diese zusätzlich durch Kleben oder Schweißen gesichert und verstärkt werden oder sein.

Bei einer weiteren Ausführungsform der erfindungsgemäßen Elektrode kann vorgesehen sein, dass der ungewendelte Elektrodenabschnitt aus einer flexiblen und in Gebrauchsstellung zu einer Röhre gerollten Leiterplattenfolie hergestellt ist. Diese Leiterplattenfolie kann zumindest eine entlang ihrer Längserstreckung, vorzugsweise parallel zu einer Längsachse der Leiterplattenfolie, verlaufende Leiterbahn als ungewendelten Leiter aufweisen. Die Verwendung von Leiterplattenfolien, die entsprechend aufgebrachte Leiterbahnen aufweisen, kann die Herstellung des ungewendelten Elektrodenabschnitts vereinfachen. Die Röhre kann einen runden, kreisrunden, eckigen oder auch mehreckigen Querschnitt aufweisen. Die Verwendung einer Röhre mit einem eckigen oder mehreckigen Querschnitt kann für eine zuverlässige Kontaktierung des wenigstens einen Kontaktelements vorteilhaft sein. Dies insbesondere dann, wenn das wenigstens eine Kontaktelement im Bereich einer Außenecke oder einer Außenkante der Röhre mit eckigem Querschnitt angeordnet ist. Hier steht das wenigstens eine Kontaktelement dann etwas über und kann zuverlässig mit dem gewendelten Leiter des gewendelten Elektrodenabschnitts kontaktiert werden.

Der ungewendelte Elektrodenabschnitt kann, insbesondere wenn dieser aus einer zu einer Röhre gerollten Leiterplattenfolie gebildet ist, in seinem Inneren ein stabilisierendes Stützelement aufweisen. Als Stützelement kann beispielsweise ein Stützschlauch verwendet werden. Alternativ oder zusätzlich dazu kann der ungewendelte Elektrodenabschnitt in seinem Inneren eine stabilisierende Füllung aufweisen. Diese Füllung kann beispielsweise aus Epoxidharz bestehen.

Insbesondere dann, wenn der ungewendelte Elektrodenabschnitt aus einer zu einer Röhre gerollten Leiterplattenfolie gebildet ist, kann als Adapter ein Adapterabschnitt der Leiterplattenfolie dienen. Damit wird bei einer solchen Elektrode zum Beispiel ein Adapter verwendet, wie er mit Anspruch 7 beansprucht wird.

Dabei kann der Adapterabschnitt für jede Leiterbahn jeweils eine mit einer Leiterbahn elektrisch verbundene Kontaktfläche als Kontaktelement aufweisen. Je nach Wicklungsrichtung der Leiterplattenfolie zur Herstellung der Röhre kann die wenigstens eine Kontaktfläche an einer Außenseite der Röhre, die dann als Steckbereich des Adapters fungiert, oder an einer Innenseite der Röhre, die dann als Buchse des Adapters fungiert, angeordnet sein. Es ist auch möglich, dass die Kontaktfläche an einer Innenwandung der Röhre angeordnet ist, die eine Buchse des Adapters für den gewendelten Elektrodenabschnitt bildendet und diese umfangsseitig begrenzt.

Um eine Befestigung eines in die Buchse des Adapters eingesteckten gewendelten Elektrodenabschnitts an der zumindest einen als Kontaktelement dienenden Kontaktfläche des Adapterabschnitts zu vereinfachen, kann der Adapterabschnitt der Leiterplattenfolie zumindest einen Durchbruch aufweisen. Durch diesen Durchbruch kann die wenigstens eine Kontaktfläche des Adapterabschnitts von außen zugänglich sein.

Eine weitere Besonderheit der Elektrode besteht darin, dass sie nahezu beliebig erweitert werden kann und einen modularen Aufbau ermöglicht. So kann die Elektrode mehrere gewendelte Elektrodenabschnitte und/oder mehrere ungewendelte Elektrodenabschnitte umfassen, wobei zwei aufeinander folgende Elektrodenabschnitte durch jeweils einen Adapter miteinander verbunden sind. Somit kann die Elektrode beispielsweise einen ersten ungewendelten Elektrodenabschnitt und einen ersten gewendelten Elektrodenabschnitt aufweisen, die untereinander mittels eines zuvor beschriebenen Adapters verbunden sind. Der erste gewendelte Elektrodenabschnitt kann dann seinerseits über einen weiteren Adapter mit einem weiteren ungewendelten Elektrodenabschnitt verbunden sein. Somit lassen sich nahezu beliebig lange elektromedizinische Elektroden mit aufeinanderfolgenden gewendelten und ungewendelten Elektrodenabschnitten zusammenstellen.

Die Elektrode kann zudem eine Schutzumhüllung aufweisen, mit der der Adapter und eine Verbindungsstelle zwischen zwei Elektrodenabschnitten umgeben sind. Mithilfe dieser Schutzumhüllung kann die Verbindung, die mithilfe des Adapters zwischen den beiden Elektrodenabschnitten hergestellt ist, stabilisiert und vor äußeren Einflüssen geschützt werden.

Zur Lösung der eingangs genannten Aufgabe wird auch ein elektromedizinischer Impulsgeber, insbesondere ein implantierbares elektromedizinisches Stimulationsgerät, vorgeschlagen, der als elektromedizinische Elektrode eine elektromedizinische Elektrode nach einem der Ansprüche 9 bis 19 aufweist.
Dabei kann der elektromedizinische Impulsgeber als Neurostimulator ausgebildet sein, wie er zum Beispiel als Hirnschrittmacher verwendet und implantiert wird.

Es zeigen in zum Teil stark schematisierter Darstellung:
- Figur 1:: eine perspektivische Darstellung eines aus einem Mehrlumenschlauch gebildeten ungewendelten Elektrodenabschnitts mit einem erfindungsgemäßen Adapter an einem Ende des ungewendelten Elektrodenabschnitts,
- Figur 2:: eine perspektivische Darstellung einer erfindungsgemäßen Elektrode, wobei ein gewendelter Elektrodenabschnitt mit insgesamt vier gewendelten elektrischen Leitern über einen Steckbereich des in Figur 1 dargestellten Adapters geschoben ist, um den gewendelten Elektrodenabschnitt mit dem in Figur 1 ebenfalls dargestellten ungewendelten Elektrodenabschnitt zu verbinden, wobei zu erkennen ist, dass jeder der gewendelten Leiter jedes Kontaktelement des Adapters zumindest einmal kontaktiert,
- Figur 3:: eine perspektivische Darstellung eines ungewendelten Elektrodenabschnitts mit einem an diesem ausgebildeten Adapterbereich, wobei der Elektrodenabschnitt und der Adapter durch Rollen einer flexiblen Leiterplattenfolie zu einem zylindrischen Hohlkörper, hier einer Röhre, gebildet sind,
- Figur 4:: eine perspektivische Darstellung einer erfindungsgemäßen Elektrode, deren gewendelter Elektrodenabschnitt mit ihren vier gewendelten elektrischen Leitern über den einen Adapter der Elektrode bildenden Adapterbereich der zu der Röhre gerollten Leiterplattenfolie des ungewendelten Elektrodenabschnitts geschoben ist, sodass die gewendelten Leiter den Adpater und den Steckbereich des Adapters mehrfach umrunden und jedes Kontaktelement des Adpaters wenigstens einmal berühren,
- Figur 5:: eine Seitenansicht einer mit der in den Figuren 1 und 2 dargestellten Elektrode vergleichbaren Elektrode mit einer geschnitten dargestellten Schutzumhüllung, die den Adapter und die Verbindungsstelle zwischen den beiden Elektrodenabschnitten umgibt, sowie
- Figur 6:: eine vergrößerte Darstellung der Verbindungsstelle zwischen den beiden Elektrodenabschnitten aus Figur 5.

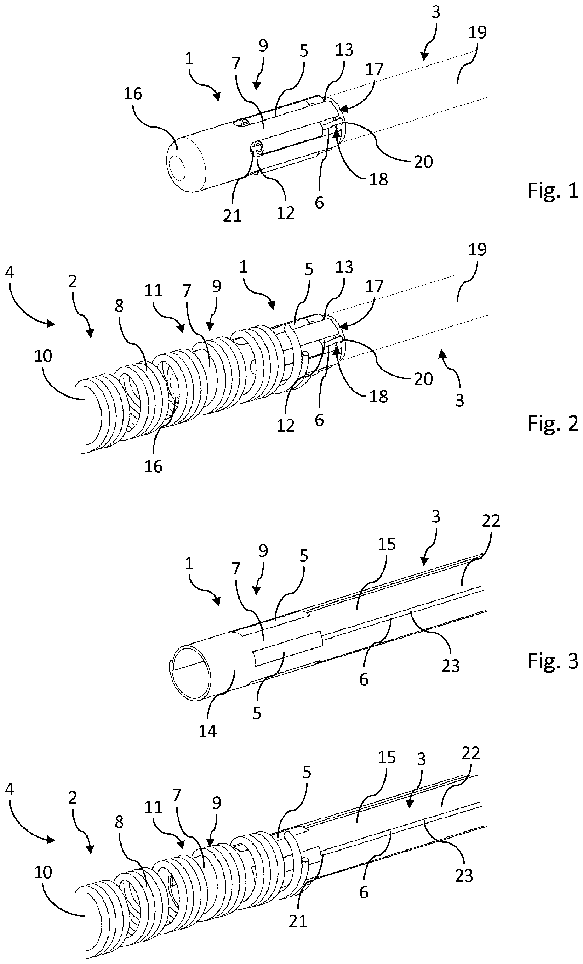

Die Figuren 1 bis 6 zeigen unterschiedliche Ausführungsformen eines elektromedizinischen Adapters. Der Adapter 1 wird im Ganzen mit 1 bezeichnet. In der nachfolgenden Beschreibung erhalten in ihrer Funktion übereinstimmende Elemente auch bei abweichender Gestaltung oder Formgebung übereinstimmende Bezugszahlen.

Die in den Figuren dargestellten elektromedizinischen Adapter 1 sind zur elektrischen Verbindung eines gewendelten Elektrodenabschnitts 2 mit einem ungewendelten Elektrodenabschnitt 3 einer im Ganzen mit 4 bezeichneten Elektrode eingerichtet. Der Adapter 1 weist dazu wenigstens ein Kontaktelement 5 auf, das mit einem ungewendelten Leiter 6 des ungewendelten Elektrodenabschnitts 3 verbindbar und derart an einem Grundkörper 7 des Adapters 1 angeordnet ist, dass ein in Gebrauchsstellung das wenigstens eine Kontaktelement 5 kontaktierender gewendelter Elektrodenabschnitt 2 eine Längsachse des Grundkörpers 7 des Adapters 1 mit seinem wenigstens einen gewendelten Leiter 8 radial umgibt.

Der in den Figuren 2, 4, 5 und 6 dargestellte gewendelte Elektrodenabschnitt 2 weist insgesamt vier gewendelte Leiter 8 auf.

Alle in den Figuren 1 bis 6 dargestellten Ausführungsformen des Adapters 1 weisen an ihren Grundkörpern 7 jeweils einen Steckabschnitt 9 auf. Die insgesamt fünf Kontaktelemente 5 des jeweiligen Adapters 1 sind an einer Außenseite des Steckabschnitts 9 angeordnet. Der jeweilige Adapter 1 kann mit seinem Steckabschnitt 9, wie es in den Figuren 2 und 4 gezeigt ist, in eine von dem wenigstens einen gewendelten Leiter 8 des gewendelten Elektrodenabschnitts 2 definierte Innenlängshöhlung 10 in ein Anschlussende 11 des gewendelten Elektrodenabschnitts 2 zur Kontaktierung des gewendelten Elektrodenabschnitts 2 und seiner gewendelten Leiter 8 eingesteckt werden.

Bei einer anderen, in den Figuren nicht dargestellten Ausführungsform des Adapters 1 ist vorgesehen, dass der Grundkörper 7 dieses Adapters 1 eine Buchse zur Aufnahme eines Anschlussendes 11 des gewendelten Elektrodenabschnitts 2 aufweist. In dieser Buchse ist dann wenigstens ein Kontaktelement 5 zur Kontaktierung des wenigstens einen gewendelten Leiters angeordnet. Sind mehrere Kontaktelemente5 vorhanden, sind diese vorzugsweise gleichmäßig verteilt um eine Längsachse des Adapters 1, seines Grundkörpers 7 oder auch seiner Buchse angeordnet.

Die Grundkörper 7 der dargestellten Adapter 1 bestehen aus einem elektrisch isolierenden Material. Der Adapter 1, der in den Figuren 1 und 2 dargestellt ist, weist an seinem Grundkörper 7 für jedes der Kontaktelemente 5 jeweils eine nutförmige Halteaufnahme 12 auf. In jede der insgesamt fünf Halteaufnahmen 12 ist jeweils ein Kontaktelement 5 eingesetzt. Jede der Halteaufnahmen 12 ist an dem Steckabschnitt 9 des Adapters 1 angeordnet.

Bei einem Adapter 1, der eine Buchse zur Aufnahme des gewendelten Elektrodenabschnitts 2 aufweist, sind die Halteaufnahmen 12 entsprechend innerhalb der Buchse angeordnet. Die Halteaufnahmen 12 und die Kontaktelemente 5 sind gleichmäßig verteilt um eine Längsachse des Grundkörpers 7 des Adapters 1 und damit so an dem Adapter 1 angeordnet, dass sie gut von den gewendelten Leitern 8 des gewendelten Elektrodenabschnitts 2 kontaktiert werden können. Hierbei fällt auf, dass die gewendelten Leiter 8 den Steckbereich 9 des Adapters 1 und die daran angeordneten Kontaktelemente 5 mehrfach, das heißt in einem Winkelbereich von mehr als 360 Grad, umrunden. Dadurch kontaktiert jeder der vier gewendelten Leiter 8 jedes der fünf Kontaktelemente 5 wenigstens einmal. Dies begünstigt eine zuverlässige elektrische Verbindung der Elektrodenabschnitte 2 und 3.

Die Halteaufnahmen 12 des Adapters 1 aus den Figuren 1 und 2 weisen stirnseitige Öffnungen 13 auf. Durch diese stirnseitigen Öffnungen 13 lassen sich zunächst die Kontaktelemente 5 in die Halteaufnahmen 12 einführen. Anschließend können auch die ungewendelten Leiter 6 des ungewendelten Elektrodenabschnitts 3 durch diese stirnseitigen Öffnungen 13 mit den Kontaktelementen 5 verbunden werden.

Bei dem Ausführungsbeispiel des Adapters 1, das in den Figuren 1 und 2 sowie 5 und 6 dargestellt ist, handelt es sich bei den Kontaktelementen 5 um Kontakthülsen, die in Gebrauchsstellung in die Halteaufnahmen 12 des Adapters 1 eingesetzt sind. Die als Kontakthülsen vorliegenden Kontaktelemente 5 ragen in ihrer Gebrauchsstellung radial nach außen aus den Halteaufnahmen 12 und stehen zur Kontaktierung der insgesamt vier gewendelten Leitern 8 des gewendelten Elektrodenabschnitts 2 nach außen über einen Außenumfang des Grundkörpers 7 des Adapters 1 über.

Der Adapter 1 weist mehrere gleichmäßig um eine Längsachse des Adapters 1 und seines Grundkörpers 7 verteilt angeordnete Kontaktelemente 5 und Halteaufnahmen 12 auf. Gemäß den Figuren 3 und 4 ist auch bei dem dort gezeigten Adapter 1 vorgesehen, dass hier mehrere Kontaktelemente 5 gleichmäßig verteilt um die Längsachse dieses Adapters 1 angeordnet sind.

Beiden Adapter 1 ist gemein, dass sie so viele Halteaufnahmen 12 bzw. Kontaktelemente 5 wie der mit dem Adapter 1 jeweils zu verbindende ungewendelte Elektrodenabschnitt 3 ungewendelte Leiter 6 aufweist. Somit steht für jeden ungewendelten Leiter 6 des ungewendelten Elektrodenabschnitts 3 jeweils ein Kontaktelement 5 und, im Falle des Adapters 1 gemäß den Figuren 1 und 2 sowie 5 und 6, auch jeweils eine Halteaufnahme 12 zur Verfügung.

Insbesondere die als Kontakthülsen ausgebildeten Kontaktelemente 5 können jeweils eine außenseitige Rastnut aufweisen. In diese Rastnut kann zumindest einer der vier gewendelten Leiter 8 des gewendelten Elektrodenabschnitts 2 bei in Gebrauchsstellung an dem Adapter 1 befindlichem gewendelten Elektrodenabschnitt 2 einrasten und in Gebrauchsstellung eingerastet sein.

Bei dem in den Figuren 3 und 4 dargestellten Ausführungsbeispiel des elektromedizinischen Adapters 1 ist der Grundkörper 7 des Adapters 1 aus einem Adapterabschnitt 14 einer flexiblen Leiterplattenfolie 15 hergestellt. Dies, indem der Adapterabschnitt 14 der flexiblen Leiterplattenfolie 15 zu einem zylindrischen Hohlkörper, hier zu einer Röhre, gerollt ist.

Die fünf Kontaktelemente 5 dieses Adapters 1 sind an dem Adapterabschnitt 14 ausgebildete Kontaktflächen. Jede der als Kontaktflächen ausgebildeten Kontaktelemente 5 ist mit jeweils einer Leiterbahn, die einen ungewendelten Leiter 6 des ungewendelten Elektrodenabschnitts 3 bildet, elektrisch verbunden.

Die Grundkörper 7 beider Adapter 1 sind zylindrisch und weisen einen runden oder kreisrunden Querschnitt auf. Der Grundkörper 7 des in den Figuren 1 und 2 dargestellten Adapters ist in seinem Steckbereich 9 an einem freien Ende, das als Einsteckende fungiert, mit einer Anfasung oder Abrundung 16 versehen. Mit Hilfe dieser Abrundung 16 kann der Adapter 1 leichter in die bereits zuvor erwähnte Innenlängshöhlung 10 des gewendelten Elektrodenabschnitts 2 eingeführt werden.

An einem der Abrundung 16 gegenüberliegenden Ende des Grundkörpers 7 des Adapters 1 weist der Grundkörper 7 ein Steckelement 17 auf. Ein dazu korrespondierend ausgebildetes Gegensteckelement 18 ist an der Stirnseite des ungewendelten Elektrodenabschnitts 3 angeordnet. So ist zwischen dem Adapter 1 und dem ungewendelten Elektrodenabschnitt 3 eine Steckverbindung vorgesehen, über die der Adapter 1 bzw. sein Grundkörper 7 mit dem ungewendelten Elektrodenabschnitt 3 verbunden werden kann.

Die elektromedizinische Elektrode 4 ist als Stimulationselektrode ausgebildet und kann beispielsweise dazu verwendet werden, Stimulationsimpulse zum Beispiel auf das Gehirn oder ein anderes Organ eines Patienten abzugeben. Die elektromedizinischen Elektroden 4, die in den Figuren 1 und 2 sowie 3 und 4 dargestellt sind, umfassen jeweils einen gewendelten Elektrodenabschnitt 2 und einen ungewendelten Elektrodenabschnitt 3 auf. Der gewendelte Elektrodenabschnitt 2 umfasst insgesamt vier gewendelte elektrische Leiter 8, die gemeinsam die bereits zuvor erwähnte Innenlängshöhlung 10 definieren und mehrfach umschließen oder umlaufen. Der ungewendelte Elektrodenabschnitt 3 umfasst zumindest einen und im vorliegenden Ausführungsbeispiel fünf ungewendelte elektrische Leiter 6. Die Leiter 6 und 8 der beiden Elektrodenabschnitte 2 sind über einen der beiden bereits zuvor beschriebenen Adapter 1 elektrisch miteinander verbunden.

Die Figuren 2 und 4 sowie 5 und 6 verdeutlichen, dass die gewendelten Leiter 8 des gewendelten Elektrodenabschnitts 2 den jeweiligen Steckbereich 9 des Adapters 1 zur Kontaktierung des ungewendelten Elektrodenabschnitts 3 mit einem Anschlussende 11 außenseitig übergreifen. Bei einer in den Figuren nicht dargestellten Ausführungsform der elektromedizinischen Elektrode 4 ist vorgesehen, dass der gewendelte Abschnitt 2 mit einem Anschlussende 11 in eine Buchse eines elektromedizinischen Adapters 1 eingesteckt und dadurch mit dem ungewendelten Elektrodenabschnitt 3 elektrisch verbunden ist.

Bei den in den Figuren 1, 2, 5 und 6 dargestellten Ausführungsbeispielen der elektromedizinischen Elektrode 4 ist der ungewendelte Elektrodenabschnitt 3 von einem sogenannten Mehrlumenschlauch 19 gebildet. Dieser umfasst für jeden der fünf ungewendelten Leiter 6 jeweils ein inneres Lumen 20, in dem jeweils ein ungewendelter Leiter 6 verläuft.

Jeder ungewendelte Leiter 6 des ungewendelten Elektrodenabschnitts 3 weist ein aus dem Lumenschlauch 19 ragendes Leiterende 21 auf. Jedes Leiterende 21 ist mit jeweils einem Kontaktelement 5 des Adapters 1 verbunden. Da die Kontaktelemente 5 des in den Figuren 1 und 2 dargestellten Adapters 1 aus Kontakthülsen gebildet sind, können die Leiterenden 21 in die Kontakthülsen 5 eingeschoben und mittels Crimpen oder Vercrimpen der Kontakthülsen 5 mit den Kontakthülsen 5 verbunden werden. Die gewendelten Leiter 8 des gewendelten Elektrodenabschnitts 2 können mit den hülsenförmigen Kontaktelementen 5 zum Beispiel durch Schweißen, Kleben, Löten, Bonden oder Drahtbonden verbunden werden.

Jeder gewendelte Leiter 8 des gewendelten Elektrodenabschnitts 2 kontaktiert alle der insgesamt fünf vorhandenen Kontaktelemente 5 des Adapters 1. Dies gilt für beide der in den Figuren 1 bis 4 dargestellten Ausführungsbeispiele der Adapter 1 und Elektroden 4.

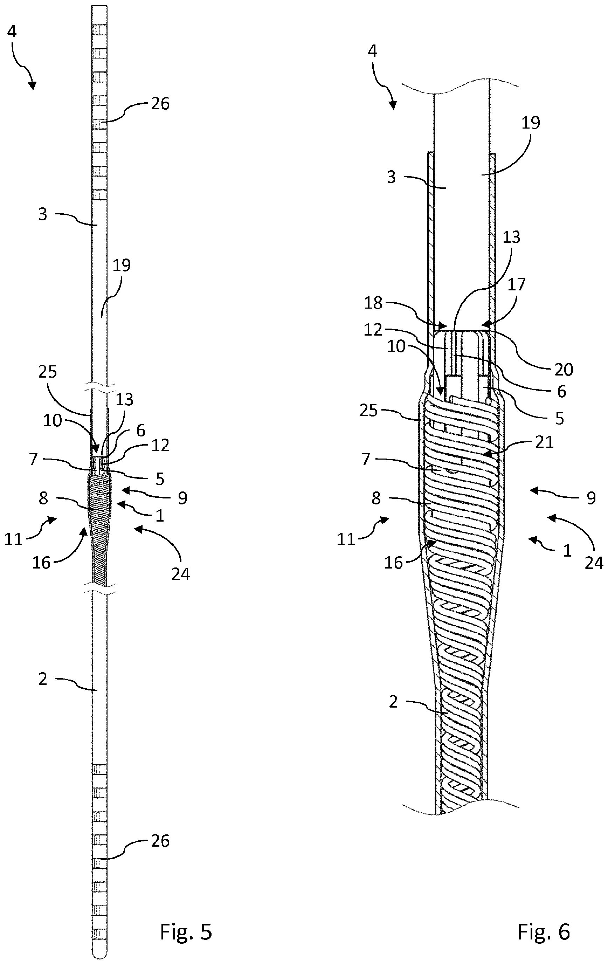

Ein lichter Innendurchmesser des gewendelten Elektrodenabschnitts 2, genauer gesagt seiner Innenlängshöhlung 10 im Bereich seines Anschlussendes 11, ist kleiner als ein Durchmesser eines gedachten Hüllkreises, der die in Gebrauchsstellung befindlichen Kontaktelemente 5 an der Außenseite des Steckbereichs 9 des jeweiligen Adapters 1 einhüllt. Dies hat zur Folge, dass der gewendelte Elektrodenabschnitt 2 den Adapter 1 und seine Kontaktelemente 5 insbesondere mit seinem Anschlussende 11 unter radialer Pressung übergreift. Dies ermöglicht eine kraftschlüssige Verbindung zwischen dem gewendelten Elektrodenabschnitt 2 und dem Adapter 1 sowie einen zuverlässigen Sitz des gewendelten Elektrodenabschnitts 2 an dem Adapter 1 und eine stabile Verbindung der beiden Elektrodenabschnitte 2 und 3. Die Aufspreizung, die der gewendelte Elektrodenabschnitt 2 im Bereich seines Anschlussendes 11 dabei erfährt, ist gut bei dem in den Figuren 5 und 6 dargestellten Ausführungsbespiel oder Elektrode 4 zu erkennen.

Bei dem Ausführungsbeispiel der Elektrode 4 gemäß den Figuren 3 und 4 ist der ungewendelte Elektrodenabschnitt 3 aus einer flexiblen und in Gebrauchsstellung zu einer Röhre 22 gerollten Leiterplattenfolie 15 hergestellt. Die Leiterplattenfolie 15 weist insgesamt fünf Leiterbahnen 23 auf, die entlang der Längserstreckung der Leiterplattenfolie 15 verlaufen und parallel zueinander und zu einer Längsachse der Leiterplattenfolie 15 ausgerichtet sind. Diese Leiterbahnen 23 fungieren als ungewendelte Leiter 6 dieser Form eines ungewendelten Elektrodenabschnitts 3.

Der ungewendelte Elektrodenabschnitt 3 kann in seinem Inneren, insbesondere dann, wenn der ungewendelte Elektrodenabschnitt 3 aus einer zu einer Röhre 22 gerollten Leiterplattenfolie 15 gebildet ist, ein stabilisierendes Stützelement und/oder eine stabilisierende Füllung, beispielsweise aus Epoxidharz, enthalten.

Wie bereits zuvor ausgeführt wurde, ist der Adapter 1 bei der in den Figuren 3 und 4 dargestellten elektromedizinischen Elektrode 4 von einem Adapterabschnitt 14 der Leiterplattenfolie 15 des ungewendelten Elektrodenabschnitts 3 gebildet. Somit wird der Adapter 1 beim Rollen der flexiblen Leiterplattenfolie 15 zur Herstellung des ungewendelten Elektrodenabschnitts 3 automatisch mit hergestellt. Der Adapterabschnitt 14 ist somit Teil der Leiterplattenfolie 15 und weist für jede Leiterbahn 23 jeweils eine mit einer Leiterbahn 23 elektrisch verbundene Kontaktfläche als Kontaktelement 5 auf. Dies ist gut in den Figuren 3 und 4 zu erkennen.

Je nach Wicklungsrichtung der flexiblen Leiterplattenfolie 15 zur Herstellung des ungewendelten Elektrodenabschnitts 3 kann diese als Kontaktelement 5 dienende Kontaktfläche dann an einer Außenseite oder auch an einer Innenseite der so hergestellten Röhre 22 angeordnet sein. Ist die zumindest eine Kontaktfläche 5 an einer Innenseite der zu einer Röhre 22 gerollten Leiterplattenfolie 15 angeordnet, erhält man einen Adapter 1 mit einer Buchse, die zur Aufnahme eines Anschlussendes 11 eines mit dem Adapter 1 zu verbindenden gewendelten Elektrodenabschnitts 2 geeignet ist.

Hier ist dann die zumindest eine Kontaktfläche 5 an einer eine Buchse des Adapters 1 zur Aufnahme des gewendelten Elektrodenabschnitts 2 begrenzenden Innenwandung der Röhre 22 angeordnet.

Die Elektrode 4 kann mehr als die in den Figuren dargestellten beiden Elektrodenabschnitte 2 und 3 umfassen. Insbesondere dort, wo ein gewendelter Elektrodenabschnitt 2 auf einen ungewendelten Elektrodenabschnitt 3 trifft oder umgekehrt, ist dann jeweils ein Adapter 1 zur Verbindung der beiden Elektrodenabschnitte 2 und 3 vorgesehen.

Bei dem in den Figuren 5 und 6 dargestellten Ausführungsbeispiel der Elektrode 4 ist eine Verbindungsstelle 24 zwischen den beiden Elektrodenabschnitten 2 und 3 von einer Schutzumhüllung 25 umgeben und durch diese zusätzlich stabilisiert. Die Schutzumhüllung 25 schützt zudem die mithilfe des Adapters 1 hergestellte elektrische Verbindung zwischen den Elektrodenabschnitten 2 und 3. An ihren der Verbindungsstelle 25 abgewandten Enden weisen beide Elektrodenabschnitte 2 und 3 mehrere Elektrodenkontakte 26 auf. Mit den Elektrodenkontakte 26 kann einer der Elektrodenabschnitte 2 oder 3 zum Beispiel in einer entsprechend ausgestalteten Anschlussbuchse eines elektromedizinischen Impulsgebers platziert und an diesen angeschlossen werden. Die an dem distalen Ende der Elektrode 4 angeordneten Elektrodenkontakte 26 des entsprechenden Elektrodenabschnitts 2 oder 3 werden dann zum Beispiel in einem Zielgebiet implantiert, auf das über die Elektrodenkontakte 26 elektrische Impulse abgegeben werden sollen.

Die in den Figuren gezeigten Bauformen der elektromedizinischen Elektrode 4 lassen sich an einem elektromedizinischen Impulsgeber einsetzen. Besonders vorteilhaft lassen sich die elektromedizinischen Elektroden 4 bei einem implantierbaren elektromedizinischen Stimulationsgerät, wie beispielsweise einem elektromedizinischen Neurostimulator, verwenden. Ein solcher Neurostimulator wird zum Beispiel zur Stimulation von bestimmten Hirnregionen eingesetzt. Dabei wird der Neurostimulator im Rumpf, insbesondere im Bereich des Schlüsselbeins oder der Brust, eines Patienten eingesetzt. Das Zielgebiet, auf das die Stimulationsimpulse mittels der Elektrode 4 abgegeben werden sollen, liegt dann im Kopf des Patienten. Um die Strecke zwischen dem Zielgebiet und der Implantationsstelle, an der der elektromedizinische Impulsgeber implantiert ist, zu überbrücken, wird die erfindungsgemäße Elektrode 4 verwendet. Hier kann in einem Streckenabschnitt, der noch im Rumpf des Patienten verläuft, ein ungewendelter Elektrodenabschnitt 3 verlegt werden. Im Rumpf ist der Elektrodenabschnitt 3 vergleichsweise geringen Bewegungen ausgesetzt, so dass hier ein ungewendelter Elektrodenabschnitt zum Einsatz kommen kann. Im Halsbereich, der naturgemäß größeren Bewegungen ausgesetzt ist als der Rumpf, kann als Elektrodenabschnitt ein gewendelter Elektrodenabschnitt 2 eingesetzt werden, der flexibler und bruchresistenter als ein ungewendelter Elektrodenabschnitt 3 ist.

Auf diese Weise wird eine auf den Anwendungsfall maßgeschneiderte Elektrode 4 verwendet. Dies mit dem Vorteil, dass bei ausreichend großer Flexibilität durch den gewendelten Elektrodenabschnitt 2 ein vergleichsweise niedriger elektrischer Gesamtwiderstand der Elektrode 4 durch den ungewendelten Elektrodenabschnitt 3 erzielt werden kann.

Die Erfindung befasst sich mit Verbesserungen auf dem technischen Gebiet der elektromedizinischen Stimulation. Hierzu werden ein elektromedizinischer Adapter 1, eine elektromedizinische Elektrode 4 und ein elektromedizinischer Impulsgeber vorgeschlagen. Um einen ungewendelten Elektrodenabschnitt 3 mit einem gewendelten Elektrodenabschnitt 2 zu verbinden, wird insbesondere der Adapter 1 vorgeschlagen, der zumindest ein Kontaktelement 5 aufweist, das von einem gewendelten Elektrodenabschnitt 2 einer Elektrode 4 derart kontaktiert wird, dass der gewendelte Elektrodenabschnitt 2 eine Längsachse des Grundkörpers 7 des Adapters 1 mit seinem wenigstens einem gewendelten Leiter 8 umschließt, also radial umgibt.

### Bezugszeichenliste

- 1: Adapter
- 2: gewendelter Elektrodenabschnitt
- 3: ungewendelter Elektrodenabschnitt
- 4: Elektrode
- 5: Kontaktelement/Kontakthülse/Kontaktfläche
- 6: ungewendelter Leiter
- 7: Grundkörper
- 8: gewendelter Leiter
- 9: Steckbereich von 1
- 10: Innenlängshöhlung von 2
- 11: Anschlussende von 2
- 12: Halteaufnahme
- 13: stirnseitige Öffnung von 12
- 14: Adapterabschnitt
- 15: Leiterplattenfolie
- 16: Abrundung
- 17: Steckelement an 1
- 18: Gegensteckelement an 19
- 19: Lumenschlauch/Mehrlumenschlauch
- 20: Lumen
- 21: Leiterende
- 22: Röhre
- 23: Leiterbahn
- 24: Verbindungsstelle
- 25: Schutzumhüllung
- 26: Elektrodenkontakte

## Patentansprüche

1. Elektromedizinischer Adapter (1) zur elektrischen Verbindung eines gewendelten Elektrodenabschnitts (2) mit einem ungewendelten Elektrodenabschnitt (3) einer elektromedizinischen Elektrode (4), wobei der Adapter (1) wenigstens ein Kontaktelement (5) aufweist, das mit einem ungewendelten Leiter (6) des ungewendelten Elektrodenabschnitts (3) verbindbar und derart an einem Grundkörper (7) des Adapters (1) angeordnet ist, dass ein in Gebrauchsstellung das wenigstens eine Kontaktelement (5) kontaktierender gewendelter Elektrodenabschnitt (2) eine Längsachse des Grundkörpers (7) des Adapters (1) mit seinem wenigstens einen gewendelten Leiter (8) umschließt, und wobei das wenigstens eine Kontaktelement (5) eine Kontakthülse ist, die in Gebrauchsstellung in eine Halteaufnahme (12) des Adapters (1) eingesetzt ist, **dadurch gekennzeichnet, dass** das wenigstens eine Kontaktelement (5) in Gebrauchsstellung zur Kontaktierung wenigstens eines gewendelten Leiters (8) eines gewendelten Elektrodenabschnitts (2) radial aus der Halteaufnahme (12) nach außen oder nach innen übersteht.

2. Elektromedizinischer Adapter (1) nach Anspruch 1, **dadurch gekennzeichnet, dass** der Grundkörper (7) des Adapters (1) einen Steckbereich (9) aufweist und dass zumindest ein Kontaktelement (5) des Adapters (1) an einer Außenseite des Steckbereichs (9) angeordnet ist, wobei der Adapter (1) mit seinem Steckbereich (9) in eine von dem wenigstens einen gewendelten Leiter (8) definierte Innenlängshöhlung (10) in einem Anschlussende (11) des gewendelten Elektrodenabschnitts (2) zur Kontaktierung des gewendelten Elektrodenabschnitts (2) einsteckbar ist, und/oder dass der Grundkörper (7) des Adapters (1) eine Buchse aufweist, in der wenigstens ein Kontaktelement (5) zur Kontaktierung des wenigstens einen gewendelten Leiters (8) angeordnet ist und die zur Aufnahme eines Anschlussendes (11) des gewendelten Elektrodenabschnitts (2) eingerichtet ist.

3. Elektromedizinischer Adapter (1) nach Anspruch 1 oder 2, **dadurch gekennzeichnet, dass** der Grundkörper (7) des Adapters (1) aus einem elektrisch isolierenden Material besteht und/oder dass der Adapter (1) an seinem Grundkörper (7) für jedes Kontaktelement (5) jeweils eine Halteaufnahme (12) aufweist, in die ein Kontaktelement (5) einsetzbar oder eingesetzt ist, insbesondere wobei wenigstens eine Halteaufnahme (12) an dem Steckbereich (9) des Adapters (1) und/oder wobei wenigstens eine Halteaufnahme (12) innerhalb der Buchse angeordnet ist/sind, vorzugsweise wobei die Halteaufnahme (12) eine stirnseitige Öffnung (13) aufweist.

4. Elektromedizinischer Adapter (1) nach einem der Ansprüche 1 bis 3, **dadurch gekennzeichnet, dass** der Adapter (1) mehrere, vorzugsweise gleichmäßig um eine Längsachse des Adapters (1) verteilt angeordnete, Kontaktelemente (5) und/oder Halteaufnahmen (12) aufweist, insbesondere wobei in jeder der Halteaufnahmen (12) jeweils ein Kontaktelement (5) anordenbar oder angeordnet ist, und/oder dass der Adapter (1) so viele Kontaktelemente (5) und/oder Halteaufnahmen (12) wie der mit dem Adapter (1) zu verbindende ungewendelte Elektrodenabschnitt (3) ungewendelte Leiter (6) aufweist.

5. Elektromedizinischer Adapter (1) nach einem der Ansprüche 1 bis 4, **dadurch gekennzeichnet, dass** der Adapter (1) zur reversibel lösbaren Verbindung, insbesondere durch Federkraft, mit einem gewendelten Elektrodenabschnitt (2) eingerichtet ist und/oder dass das wenigstens eine Kontaktelement (5), insbesondere die Kontakthülse, eine Rastnut aufweist, in die wenigstens ein gewendelter Leiter (8) des gewendelten Elektrodenabschnitts (2) bei in Gebrauchsstellung an dem Adapter (1) befindlichem gewendelten Elektrodenabschnitt (2) einrastbar oder eingerastet ist.

6. Elektromedizinischer Adapter (1) nach einem der Ansprüche 1 bis 5, **dadurch gekennzeichnet, dass** der Grundkörper (7) des Adapters (1) aus einem zu einem Hohlkörper, insbesondere zu einer Röhre, gerollten Adapterabschnitt (14) einer Leiterplattenfolie (15) hergestellt ist, wobei das wenigstens eine Kontaktelement (5) eine an dem Adapterabschnitt (14) ausgebildete Kontaktfläche ist, insbesondere wobei der Hohlkörper einen runden, kreisrunden, eckigen oder mehreckigen Querschnitt aufweist.

7. Elektromedizinischer Adapter (1) nach einem der Ansprüche 1 bis 6, **dadurch gekennzeichnet, dass** der Grundkörper (7) des Adapters (1) zylindrisch ist und/oder einen runden oder kreisrunden Querschnitt hat, insbesondere in seinem Steckbereich (9), und/oder dass der Grundkörper (7) an einem freien Ende des Steckbereichs (9) eine Anfasung oder Abrundung (16) zum erleichterten Einführen des Grundkörpers (7) in eine Innenlängshöhlung (10) eines gewendelten Elektrodenabschnitts (2) aufweist und/oder dass der Adapter (1) an seinem Grundkörper (7) ein zu einem Gegensteckelement (18) eines ungewendelten Elektrodenabschnitts (3) korrespondierendes Steckelement (17) aufweist und in Gebrauchsstellung über eine Steckverbindung mit dem ungewendelten Elektrodenabschnitt (2) verbunden ist.

8. Elektromedizinische Elektrode (4), insbesondere Stimulationselektrode, mit wenigstens einem gewendelten Elektrodenabschnitt (2) und wenigstens einem ungewendelten Elektrodenabschnitt (3), wobei der gewendelte Elektrodenabschnitt (2) zumindest einen gewendelten elektrischen Leiter (8) und der ungewendelte Elektrodenabschnitt (3) zumindest einen ungewendelten elektrischen Leiter (6) umfassen, und wobei die Leiter (6,8) der beiden Elektrodenabschnitte (2,3) über einen Adapter (1) elektrisch miteinander verbunden sind, wobei der Adapter (1) ein Adapter (1) nach einem der Ansprüche 1 bis 7 ist.

9. Elektromedizinische Elektrode (4), insbesondere Stimulationselektrode, mit wenigstens einem gewendelten Elektrodenabschnitt (2) und wenigstens einem ungewendelten Elektrodenabschnitt (3), wobei der gewendelte Elektrodenabschnitt (2) zumindest einen gewendelten elektrischen Leiter (8) und der ungewendelte Elektrodenabschnitt (3) zumindest einen ungewendelten elektrischen Leiter (6) umfassen, und wobei die Leiter (6,8) der beiden Elektrodenabschnitte (2,3) über einen Adapter (1) elektrisch miteinander verbunden sind, **dadurch gekennzeichnet, dass** der Adapter (1) wenigstens ein Kontaktelement (5) aufweist, das mit dem zumindest einen ungewendelten Leiter (6) des ungewendelten Elektrodenabschnitts (3) verbunden und derart an einem Grundkörper (7) des Adapters (1) angeordnet ist, dass der das wenigstens eine Kontaktelement (5) kontaktierende gewendelte Elektrodenabschnitt (2) eine Längsachse des Grundkörpers (7) des Adapters (1) mit seinem wenigstens einen gewendelten Leiter (8) umschließt, dass der Grundkörper (7) des Adapters (1) einen Steckbereich (9) aufweist und dass zumindest ein Kontaktelement (5) des Adapters (1) an einer Außenseite des Steckbereichs (9) angeordnet ist, wobei der Adapter (1) mit seinem Steckbereich (9) in eine von dem wenigstens einen gewendelten Leiter (8) definierte Innenlängshöhlung (10) in einem Anschlussende (11) des gewendelten Elektrodenabschnitts (2) zur Kontaktierung des gewendelten Elektrodenabschnitts (2) eingesteckt ist, wobei der zumindest eine gewendelte Leiter (8) des gewendelten Elektrodenabschnitts (2) den Steckbereich (9) des Adapters (1) zur Kontaktierung des ungewendelten Elektrodenabschnitts (3) außenseitig übergreift.

10. Elektromedizinische Elektrode (4) nach Anspruch 8 oder 9, **dadurch gekennzeichnet, dass** der gewendelte Elektrodenabschnitt (2) reversibel lösbar mit dem Adapter (1) verbindbar und in Gebrauchsstellung verbunden ist.

11. Elektromedizinische Elektrode (4) nach Anspruch 8 oder nach dem auf Anspruch 8 rückbezogenen Anspruch 10, **dadurch gekennzeichnet, dass** der zumindest eine gewendelte Leiter (8) des gewendelten Elektrodenabschnitts (2) einen oder den Steckbereich (9) des Adapters (1) zur Kontaktierung des ungewendelten Elektrodenabschnitts (3) außenseitig übergreift und/oder dass der gewendelte Elektrodenabschnitt (2) zur Kontaktierung des ungewendelten Elektrodenabschnitts (3) mit seinem Anschlussende (11) in eine oder die Buchse des Adapters (1) eingesteckt ist.

12. Elektromedizinische Elektrode (4) nach einem der Ansprüche 8 bis 11, **dadurch gekennzeichnet, dass** der ungewendelte Elektrodenabschnitt (3) von einem Lumenschlauch, insbesondere einem Mehrlumenschlauch (19), mit zumindest einem inneren Lumen (20) und zumindest einem darin angeordneten ungewendelten Leiter (6) gebildet ist.

13. Elektromedizinische Elektrode (4) nach einem der Ansprüche 8 bis 12, **dadurch gekennzeichnet, dass** jeder ungewendelte Leiter (6) des ungewendelten Elektrodenabschnitts (3) ein aus dem Lumenschlauch (19) ragendes Leiterende (21) aufweist, das mit einem, vorzugsweise mit jeweils einem, Kontaktelement (5) des Adapters (1) verbunden ist, insbesondere durch Crimpen und/oder durch Schweißen und/oder durch Kleben und/oder durch Löten und/oder durch Bonden oder Drahtbonden und/oder durch Federkraft, und/oder dass der wenigstens eine gewendelte Leiter (8) des gewendelten Elektrodenabschnitts (2) durch Schweißen, Kleben und/oder Löten und/oder durch Bonden oder Drahtbonden und/oder durch Federkraft mit einem, vorzugsweise mit jeweils einem Kontaktelement (5) verbunden ist.

14. Elektromedizinische Elektrode (4) nach einem der Ansprüche 8 bis 13, **dadurch gekennzeichnet, dass** jeder gewendelte Leiter (8) des gewendelten Elektrodenabschnitts (2) jeweils wenigstens zwei Kontaktelemente (5) des Adapters (1) in Gebrauchsstellung kontaktiert, vorzugsweise wobei jeder gewendelte Leiter (8) des gewendelten Elektrodenabschnitts (2) alle Kontaktelemente (5) des Adapters (1) kontaktiert.

15. Elektromedizinische Elektrode (1) nach einem der Ansprüche 8 bis 14, **dadurch gekennzeichnet, dass** ein Außendurchmesser des gewendelten Elektrodenabschnitts (2) größer als ein Durchmesser eines gedachten Hüllkreises ist, der die in Gebrauchsstellung an dem Adapter (1) innerhalb seiner Buchse befindlichen Kontaktelemente (5) einhüllt, sodass der gewendelte Elektrodenabschnitt (2) unter radialer Pressung zwischen den Kontaktelementen (5) innerhalb der Buchse des Adapters angeordnet ist, oder dass ein lichter Innendurchmesser des gewendelten Elektrodenabschnitts (2) kleiner als ein Durchmesser eines gedachten Hüllkreises ist, der die in Gebrauchsstellung befindlichen Kontaktelemente (5) einhüllt, sodass der gewendelte Elektrodenabschnitt (2) den Adapter (1) und die Kontaktelemente (5) unter radialer Pressung übergreift.

16. Elektromedizinische Elektrode (4) nach einem der Ansprüche 8 bis 15, **dadurch gekennzeichnet, dass** der ungewendelte Elektrodenabschnitt (3) aus einer flexiblen und in Gebrauchsstellung zu einer Röhre (22) gerollten Leiterplattenfolie (15) hergestellt ist, die zumindest eine, entlang der Längserstreckung der Leiterplattenfolie (15) verlaufende Leiterbahn (23) als ungewendelten Leiter (3) aufweist, vorzugsweise wobei die Röhre (22) einen runden, kreisrunden, eckigen oder mehreckigen Querschnitt aufweist.

17. Elektromedizinische Elektrode (4) nach einem der Ansprüche 8 bis 16, **dadurch gekennzeichnet, dass** der ungewendelte Elektrodenabschnitt (3), insbesondere wenn dieser aus einer zu einer Röhre (22) gerollten Leiterplattenfolie (15) gebildet ist, in seinem Inneren ein stabilisierendes Stützelement, insbesondere ein Stützschlauch, und/oder eine stabilisierende Füllung aufweist, insbesondere aus Epoxidharz.

18. Elektromedizinische Elektrode (4) nach einem der Ansprüche 8 bis 17, **dadurch gekennzeichnet, dass** der Adapter (1) von einem Adapterabschnitt (14) der Leiterplattenfolie (15) des ungewendelten Elektrodenabschnitts (3) gebildet ist, wobei der Adapterabschnitt (14) für jede Leiterbahn (23) jeweils eine mit einer Leiterbahn (23) elektrisch verbundene Kontaktfläche als Kontaktelement (5) aufweist, und/oder dass die wenigstens eine Kontaktfläche (5), je nach Wicklungsrichtung der Leiterplattenfolie (15) zur Herstellung der Röhre (22), an einer Außenseite der Röhre (22) oder an einer eine Buchse des Adapters (1) für den gewendelten Elektrodenabschnitt (2) begrenzenden Innenwandung der Röhre (22) angeordnet ist.

19. Elektromedizinische Elektrode (4) nach einem der Ansprüche 8 bis 18, **dadurch gekennzeichnet, dass** die Elektrode (4) zumindest zwei gewendelte Elektrodenabschnitte (2) und/oder zumindest zwei ungewendelte Elektrodenabschnitte (3) umfasst, wobei aufeinanderfolgende gewendelte Elektrodenabschnitte (2) und ungewendelte Elektrodenabschnitten (3) durch jeweils einen Adapter (1) miteinander verbunden sind.

20. Elektromedizinischer Impulsgeber, insbesondere implantierbares elektromedizinisches Stimulationsgerät, mit einer elektromedizinischen Elektrode (4) nach einem der Ansprüche 8 bis 19.

## Claims

1. Electromedical adapter (1) for the electrical connection of a coiled electrode portion (2) to an uncoiled electrode portion (3) of an electromedical electrode (4), wherein the adapter (1) has at least one contact element (5) which is connectable to an uncoiled conductor (6) of the uncoiled electrode portion (3) and is arranged on a base body (7) of the adapter (1) in such a way that a coiled electrode portion (2) contacting the at least one contact element (5) in a position of use encloses a longitudinal axis of the base body (7) of the adapter (1) with its at least one coiled conductor (8), and wherein the at least one contact element (5) is a contact sleeve which, in the position of use, is inserted into a retaining seat (12) of the adapter (1), **characterized in that** the at least one contact element (5) in the position of use protrudes radially outwards or inwards from the retaining seat (12) for contacting at least one coiled conductor (8) of a coiled electrode portion (2).

2. Electromedical adapter (1) according to Claim 1, **characterized in that** the base body (7) of the adapter (1) has a plug-in region (9), and **in that** at least one contact element (5) of the adapter (1) is arranged on an outer side of the plug-in region (9), wherein the adapter (1) can be plugged with its plug-in region (9) into an inner longitudinal cavity (10), defined by the at least one coiled conductor (8), in an attachment end (11) of the coiled electrode portion (2) for contacting the coiled electrode portion (2), and/or **in that** the base body (7) of the adapter (1) has a socket in which at least one contact element (5) for contacting the at least one coiled conductor (8) is arranged and which is configured to receive an attachment end (11) of the coiled electrode portion (2).

3. Electromedical adapter (1) according to Claim 1 or 2, **characterized in that** the base body (7) of the adapter (1) is made of an electrically insulating material and/or **in that** the adapter (1) has on its base body (7), for each contact element (5), a respective retaining seat (12) into which a contact element (5) is insertable or inserted, in particular wherein at least one retaining seat (12) is arranged on the plug-in region (9) of the adapter (1), and/or wherein at least one retaining seat (12) is arranged within the socket, preferably wherein the retaining seat (12) has an end-face opening (13).

4. Electromedical adapter (1) according to one of Claims 1 to 3, **characterized in that** the adapter (1) has a plurality of contact elements (5) and/or retaining seats (12), preferably distributed uniformly about a longitudinal axis of the adapter (1), in particular wherein a contact element (5) is arrangeable or arranged in each of the retaining seats (12), and/or **in that** the adapter (1) has as many contact elements (5) and/or retaining seats (12) as the uncoiled electrode portion (3) to be connected to the adapter (1) has uncoiled conductors (6) .

5. Electromedical adapter (1) according to one of Claims 1 to 4, **characterized in that** the adapter (1) is designed for reversibly detachable connection, in particular by spring force, to a coiled electrode portion (2), and/or **in that** the at least one contact element (5), in particular the contact sleeve, has a latching groove into which at least one coiled conductor (8) of the coiled electrode portion (2) is latchable or latched when the coiled electrode portion (2) is located in the position of use on the adapter (1).

6. Electromedical adapter (1) according to one of Claims 1 to 5, **characterized in that** the base body (7) of the adapter (1) is produced from an adapter portion (14) of a printed circuit board film (15) that is rolled up to form a hollow body, in particular to form a pipe, wherein the at least one contact element (5) is a contact surface formed on the adapter portion (14), in particular wherein the hollow body has a round, circular, angular or polygonal cross section.

7. Electromedical adapter (1) according to one of Claims 1 to 6, **characterized in that** the base body (7) of the adapter (1) is cylindrical and/or has a round or circular cross section, in particular in its plug-in region (9), and/or **in that** the base body (7), at a free end of the plug-in region (9), has a chamfer or rounding (16) for facilitated insertion of the base body (7) into an inner longitudinal cavity (10) of a coiled electrode portion (2), and/or **in that** the adapter (1) has, on its base body (7), a plug element (17) corresponding to a mating plug element (18) of an uncoiled electrode portion (3) and, in the position of use, is connected to the uncoiled electrode portion (2) via a plug connection.

8. Electromedical electrode (4), in particular a stimulation electrode, having at least one coiled electrode portion (2) and at least one uncoiled electrode portion (3), wherein the coiled electrode portion (2) comprises at least one coiled electrical conductor (8) and the uncoiled electrode portion (3) comprises at least one uncoiled electrical conductor (6), and wherein the conductors (6, 8) of the two electrode portions (2, 3) are electrically connected to each other via an adapter (1), wherein the adapter (1) is an adapter (1) according to one of Claims 1 to 7.

9. Electromedical electrode (4), in particular a stimulation electrode, having at least one coiled electrode portion (2) and at least one uncoiled electrode portion (3), wherein the coiled electrode portion (2) comprises at least one coiled electrical conductor (8) and the uncoiled electrode portion (3) comprises at least one uncoiled electrical conductor (6), and wherein the conductors (6, 8) of the two electrode portions (2, 3) are electrically connected to each other via an adapter (1), **characterized in that** the adapter (1) has at least one contact element (5) which is connected to the at least one uncoiled conductor (6) of the uncoiled electrode portion (3) and is arranged on a base body (7) of the adapter (1) such that the coiled electrode portion (2) contacting the at least one contact element (5) encloses a longitudinal axis of the base body (7) of the adapter (1) with its at least one coiled conductor (8), **in that** the base body (7) of the adapter (1) has a plug-in region (9), and **in that** at least one contact element (5) of the adapter (1) is arranged on an outer side of the plug-in region (9), wherein the adapter (1) can be plugged with its plug-in region (9) into an inner longitudinal cavity (10), defined by the at least one coiled conductor (8), in an attachment end (11) of the coiled electrode portion (2) for contacting the coiled electrode portion (2), wherein the at least one coiled conductor (8) of the coiled electrode portion (2) engages externally over the plug-in region (9) of the adapter (1) for contacting the uncoiled electrode portion (3).

10. Electromedical electrode (4) according to Claim 8 or 9, **characterized in that** the coiled electrode portion (2) is connectable, and in the position of use connected, in a reversibly detachable manner to the adapter (1).

11. Electromedical electrode (4) according to Claim 8, or according to Claim 10 referred back to Claim 8, **characterized in that** the at least one coiled conductor (8) of the coiled electrode portion (2) engages externally over a or the plug-in region (9) of the adapter (1) for contacting the uncoiled electrode portion (3), and/or **in that** the coiled electrode portion (2) for contacting the uncoiled electrode portion (3) is plugged with its attachment end (11) into a or the socket of the adapter (1).

12. Electromedical electrode (4) according to one of Claims 8 to 11, **characterized in that** the uncoiled electrode portion (3) is formed by a lumen tube, in particular a multi-lumen tube (19), with at least one inner lumen (20) and at least one uncoiled conductor (6) arranged therein.

13. Electromedical electrode (4) according to one of Claims 8 to 12, **characterized in that** each uncoiled conductor (6) of the uncoiled electrode portion (3) has a conductor end (21) which protrudes from the lumen tube (19) and which is connected preferably in each case to a respective contact element (5) of the adapter (1), in particular by crimping and/or by welding and/or by gluing and/or by soldering and/or by bonding or wire bonding and/or by spring force, and/or **in that** the at least one coiled conductor (8) of the coiled electrode portion (2) is connected preferably in each case to a respective contact element (5) by welding, gluing and/or soldering and/or by bonding or wire bonding and/or by spring force.

14. Electromedical electrode (4) according to one of Claims 8 to 13, **characterized in that** each coiled conductor (8) of the coiled electrode portion (2) contacts at least two contact elements (5) of the adapter (1) in the position of use, preferably wherein each coiled conductor (8) of the coiled electrode portion (2) contacts all the contact elements (5) of the adapter (1).

15. Electromedical electrode (1) according to one of Claims 8 to 14, **characterized in that** an external diameter of the coiled electrode portion (2) is greater than a diameter of an imaginary enveloping circle which envelops the contact elements (5) located in the position of use on the adapter (1), inside the socket thereof, such that the coiled electrode portion (2) is arranged with radial pressure between the contact elements (5) inside the socket of the adapter, or **in that** a clear internal diameter of the coiled electrode portion (2) is smaller than a diameter of an imaginary enveloping circle which envelops the contact elements (5) located in the position of use, such that the coiled electrode portion (2) engages with radial pressure over the adapter (1) and the contact elements (5).

16. Electromedical electrode (4) according to one of Claims 8 to 15, **characterized in that** the uncoiled electrode portion (3) is produced from a flexible printed circuit board film (15) which is rolled up in the position of use to form a pipe (22) and which has, as uncoiled conductor (3), at least one conductor track (23) extending along the longitudinal extent of the printed circuit board film (15), preferably wherein the pipe (22) has a round, circular, angular or polygonal cross section.

17. Electromedical electrode (4) according to one of Claims 8 to 16, **characterized in that** the uncoiled electrode portion (3), in particular when formed from a printed circuit board film (15) rolled up to form a pipe (22), has in its interior a stabilizing support element, in particular a support tube, and/or a stabilizing filler, in particular of epoxy resin.

18. Electromedical electrode (4) according to one of Claims 8 to 17, **characterized in that** the adapter (1) is formed from an adapter portion (14) of the printed circuit board film (15) of the uncoiled electrode portion (3), wherein the adapter portion (14) has, as contact element (5) for each conductor track (23), a contact surface electrically connected to a conductor track (23), and/or **in that** the at least one contact surface (5), depending on the winding direction of the printed circuit board film (15) for producing the pipe (22), is arranged on an outer side of the pipe (22) or on an inner wall of the pipe (22) delimiting a socket of the adapter (1) for the coiled electrode portion (2).

19. Electromedical electrode (4) according to one of Claims 8 to 18, **characterized in that** the electrode (4) comprises at least two coiled electrode portions (2) and/or at least two uncoiled electrode portions (3), wherein successive coiled electrode portions (2) and uncoiled electrode portions (3) are connected to each other by in each case one adapter (1) .

20. Electromedical pulse generator, in particular an implantable electromedical stimulation device, having an electromedical electrode (4) according to one of Claims 8 to 19.

## Revendications

1. Adaptateur électro-médical (1) pour relier électriquement un segment d'électrode spiralé (2) avec un segment d'électrode non spiralé (3) d'une électrode électro-médicale (4), l'adaptateur (1) présentant au moins un élément de contact (5) qui peut être relié avec un conducteur non spiralé (6) du segment d'électrode non spiralé (3) et est disposé sur un corps principal (7) de l'adaptateur (1) de telle sorte qu'un segment d'électrode spiralé (2) en contact en position d'utilisation avec l'au moins un élément de contact (5) entoure un axe longitudinal du corps principal (7) de l'adaptateur (1) avec son au moins un conducteur spiralé (8), et l'au moins un élément de contact (5) étant une douille de contact qui est insérée en position d'utilisation dans un logement de maintien (12) de l'adaptateur (1), **caractérisé en ce qu'**en position d'utilisation, l'au moins un élément de contact (5) dépasse radialement du logement de maintien (12) vers l'extérieur ou vers l'intérieur pour entrer en contact avec au moins un segment d'électrode spiralé (2).

2. Adaptateur électro-médical (1) selon la revendication 1, **caractérisé en ce que** le corps principal (7) de l'adaptateur (1) présente une zone d'enfichage (9) et qu'au moins un élément de contact (5) de l'adaptateur (1) est disposé sur une face extérieure de la zone d'enfichage (9), l'adaptateur (1) pouvant être enfiché avec sa zone d'enfichage (9) dans un orifice longitudinal intérieur (10) défini par l'au moins un conducteur spiralé (8) dans une extrémité de raccordement (11) du segment d'électrode spiralé (2) pour entrer en contact avec le segment d'électrode spiralé (2), et/ou que le corps principal (7) de l'adaptateur (1) présente une prise dans laquelle est disposé au moins un élément de contact (5) pour établir le contact avec l'au moins un conducteur spiralé (8) et qui est aménagée pour recevoir une extrémité de raccordement (11) du segment d'électrode spiralé (2).

3. Adaptateur électro-médical (1) selon la revendication 1 ou 2, **caractérisé en ce que** le corps principal (7) de l'adaptateur (1) est constitué d'un matériau isolant électriquement et/ou que l'adaptateur (1) présente sur son corps principal (7) un logement de maintien (12) pour chaque élément de contact (5) dans lequel un élément de contact (5) peut être inséré ou est inséré, en particulier au moins un logement de maintien (12) étant disposé sur la zone d'enfichage (9) de l'adaptateur (1) et/ou un logement de maintien (12) étant disposé à l'intérieur de la prise, le logement de maintien (12) présentant de préférence une ouverture frontale (13).

4. Adaptateur électro-médical (1) selon une des revendications 1 à 3, **caractérisé en ce que** l'adaptateur (1) présente plusieurs éléments de contact (5) et/ou logements de maintien (12) répartis de préférence régulièrement autour de l'axe longitudinal de l'adaptateur (1), un élément de contact (5) pouvant être disposé ou étant disposé en particulier dans chacun des logements de maintien (12), et/ou que l'adaptateur (1) présente autant d'éléments de contact (5) et/ou de logements de maintien (12) que le segment d'électrode non spiralé (3) à relier avec l'adaptateur (1) présente de conducteurs non spiralés (6).

5. Adaptateur électro-médical (1) selon une des revendications 1 à 4, **caractérisé en ce que** l'adaptateur (1) est aménagé pour établir une liaison réversible, en particulier par une force élastique, avec un segment d'électrode spiralé (2) et/ou que l'au moins un élément de contact (5), en particulier la douille de contact, présente une rainure d'encliquetage dans laquelle au moins un conducteur spiralé (8) du segment d'électrode spiralé (2) peut être encliqueté ou est encliqueté lorsque le segment d'électrode spiralé (2) est en position d'utilisation sur l'adaptateur (1).

6. Adaptateur électro-médical (1) selon une des revendications 1 à 5, caractérisé en ce le corps principal (7) de l'adaptateur (1) est fabriqué à partir d'un segment d'adaptateur (14) d'une feuille de circuit imprimé (15) enroulé en forme de corps creux, en particulier de tube, l'au moins un élément de contact (5) étant une surface de contact formée sur le segment d'adaptateur (14), le corps creux présentant en particulier une section ronde, circulaire, carrée ou polygonale.

7. Adaptateur électro-médical (1) selon une des revendications 1 à 6, **caractérisé en ce que** le corps principal (7) de l'adaptateur (1) est cylindrique et/ou a une section ronde ou circulaire, en particulier dans sa zone d'enfichage (9), et/ou que le corps principal (7) présente à une extrémité libre de la zone d'enfichage (9) un biseau ou un arrondi (16) pour faciliter l'insertion du corps principal (7) dans un orifice longitudinal intérieur (10) d'un segment d'électrode spiralé (2) et/ou que l'adaptateur (1) présente sur un corps principal (7) un élément d'enfichage (17) correspondant à un élément d'enfichage opposé (18) d'un segment d'électrode non spiralé (3) et est relié en position d'utilisation par une connexion enfichable avec le segment d'électrode non spiralé (2).

8. Electrode électro-médicale (4), en particulier électrode de stimulation, avec au moins un segment d'électrode spiralé (2) et au moins un segment d'électrode non spiralé (3), dans laquelle le segment d'électrode spiralé (2) comprend au moins un conducteur électrique spiralé (8) et le segment d'électrode non spiralé (3) au moins un conducteur électrique non spiralé (6), et dans laquelle les conducteurs (6, 8) des deux segments d'électrode (2, 3) sont reliés électriquement entre eux par un adaptateur (1), l'adaptateur (1) étant un adaptateur (1) selon une des revendications 1 à 7.

9. Electrode électro-médicale (4), en particulier électrode de stimulation, avec au moins un segment d'électrode spiralé (2) et au moins un segment d'électrode non spiralé (3), dans laquelle le segment d'électrode spiralé (2) comprend au moins un conducteur électrique spiralé (8) et le segment d'électrode non spiralé (3) au moins un conducteur électrique non spiralé (6), et dans laquelle les conducteurs (6, 8) des deux segments d'électrode (2, 3) sont reliés électriquement entre eux par un adaptateur (1), **caractérisée en ce que** l'adaptateur (1) présente au moins un élément de contact (5) qui est relié avec l'au moins conducteur non spiralé (6) du segment d'électrode non spiralé (3) et est disposé sur un corps principal (7) de l'adaptateur (1) de telle sorte que le segment d'électrode spiralé (2) en contact avec l'au moins un élément de contact (5) entoure un axe longitudinal du corps principal (7) de l'adaptateur (1) avec son au moins un conducteur spiralé (8), que le corps principal (7) de l'adaptateur (1) présente une zone d'enfichage (9) et que l'au moins un élément de contact (5) de l'adaptateur (1) est disposé sur une face extérieure de la zone d'enfichage (9), l'adaptateur (1) étant enfiché avec sa zone d'enfichage (9) dans un orifice longitudinal intérieur (10) défini par l'au moins un conducteur spiralé (8) dans une extrémité de raccordement (11) du segment d'électrode spiralé (2) pour entrer en contact avec le segment d'électrode spiralé, l'au moins un conducteur spiralé (8) du segment d'électrode spiralé (2) recouvrant extérieurement la zone d'enfichage (9) de l'adaptateur (1) pour entrer en contact avec le segment d'électrode non spiralé (3).

10. Electrode électro-médicale (4) selon la revendication 8 ou 9, **caractérisée en ce que** le segment d'électrode spiralé (2) peut être relié de façon réversible et est relié en position d'utilisation avec l'adaptateur (1).

11. Electrode électro-médicale (4) selon la revendication 8 ou selon la revendication 10 faisant référence à la revendication 8, **caractérisée en ce que** l'au moins un conducteur spiralé (8) du segment d'électrode spiralé (2) recouvre extérieurement une ou la zone d'enfichage (9) de l'adaptateur (1) pour entrer en contact avec le segment d'électrode non spiralé (3) et/ou que le segment d'électrode spiralé (2) est enfiché avec son extrémité de raccordement (11) dans une ou la prise de l'adaptateur (1) pour entrer en contact avec le segment d'électrode non spiralé (3).

12. Electrode électro-médicale (4) selon une des revendications 8 à 11, **caractérisée en ce que** le segment d'électrode non spiralé (3) est formé d'un tuyau à lumière, en particulier d'un tuyau à lumières multiples (19), avec au moins une lumière intérieure (20) et au moins un conducteur non spiralé (6) disposé à l'intérieur.

13. Electrode électro-médicale (4) selon une des revendications 8 à 12, **caractérisée en ce que** chaque conducteur non spiralé (6) du segment d'électrode non spiralé (3) présente une extrémité de conducteur (21) sortant du tuyau à lumière (19), lesquelles sont reliées avec un, de préférence chacune avec un élément de contact (5) de l'adaptateur (1), en particulier par sertissage et/ou par soudage et/ou par collage et/ou par brasage et/ou par pontage ou pontage par fil et/ou par une force élastique, et/ou que le ou les conducteurs spiralés (8) du segment d'électrode spiralé (2) sont reliés par soudage, collage et/ou brasage et/ou par pontage ou pontage par fil et/ou par une force élastique avec un, de préférence chacun avec un élément de contact (5).

14. Electrode électro-médicale (4) selon une des revendications 8 à 13, **caractérisée en ce que** chaque conducteur spiralé (8) du segment d'électrode spiralé (2) est en contact avec au moins deux éléments de contact (5) de l'adaptateur (1) en position d'utilisation, chaque conducteur spiralé (8) du segment d'électrode spiralé (2) étant de préférence en contact avec tous les éléments de contact (5) de l'adaptateur.

15. Electrode électro-médicale (4) selon une des revendications 8 à 14, **caractérisée en ce qu'**un diamètre extérieur du segment d'électrode spiralé (2) est supérieur à un diamètre d'un cercle inscrit imaginaire qui enveloppe les éléments de contact (5) se trouvant en position d'utilisation sur l'adaptateur (1) à l'intérieur de sa prise, de sorte que le segment d'électrode spiralé (2) est disposé sous pression radiale entre les éléments de contact (5) à l'intérieur de la prise de l'adaptateur, ou qu'un diamètre intérieur libre du segment d'électrode spiralé (2) est inférieur à un diamètre d'un cercle inscrit imaginaire qui enveloppe les éléments de contact (5) se trouvant en position d'utilisation, de sorte que le segment d'électrode spiralé (2) recouvre l'adaptateur (1) et les éléments de contact (5) sous pression radiale.

16. Electrode électro-médicale (4) selon une des revendications 8 à 15, **caractérisée en ce que** le segment d'électrode non spiralé (3) est fabriqué à partir d'une feuille de circuit imprimé (15) flexible et, en position d'utilisation, enroulée en forme de tube (22), qui présente au moins une piste conductrice (23) courant sur la longueur de la feuille de circuit imprimé (15) en guise de conducteur non spiralé (3), le tube (22) présentant de préférence une section ronde, circulaire, carrée ou polygonale.

17. Electrode électro-médicale (4) selon une des revendications 8 à 16, **caractérisée** en ce le segment d'électrode non spiralé (3), en particulier lorsque celui-ci est formé d'une feuille de circuit imprimé (15) enroulée en forme de tube (22), possède à l'intérieur un élément de support stabilisant, en particulier un tuyau de support et/ou un remplissage stabilisant, en particulier en résine époxy.

18. Electrode électro-médicale (4) selon une des revendications 8 à 17, **caractérisée en ce que** l'adaptateur (1) est formé d'un segment d'adaptateur (14) de la feuille de circuit imprimé (15) du segment d'électrode non spiralé (3), le segment d'adaptateur (14) possédant pour chaque piste conductrice (23) une surface de contact reliée électriquement avec une piste conductrice (23) en guise d'élément de contact (5), et/ou que l'au moins une surface de contact (5), selon le sens d'enroulement de la feuille de circuit imprimé (15) pour fabriquer le tube (22), est disposée sur une face extérieure du tube (22) ou sur une paroi intérieure du tube (22) délimitant un tuyau de l'adaptateur (1) pour le segment d'électrode spiralé (2).

19. Electrode électro-médicale (4) selon une des revendications 8 à 18, **caractérisée en ce que** l'électrode (4) comprend au moins deux segments d'électrode spiralés (2) et/ou au moins deux segments d'électrode non spiralés (3), les segments d'électrode spiralés (2) et les segments d'électrode non spiralés (3) successifs étant reliés entre eux par un adaptateur (1).

20. Générateur d'impulsions électro-médical, en particulier stimulateur électro-médical implantable, avec une électrode électro-médicale (4) selon une des revendications 8 à 19.
